# EUROPEAN PATENT SPECIFICATION

(11) **EP 1 501 531 B1**
(45) Date of publication and mention of the grant of the patent: **07.01.2009**
(21) Application number: 03719604.5
(22) Date of filing: 17.04.2003
(51) Int. Cl.: A61K 39/395, A61P 25/22, A61P 25/28, C07K 16/18

(54) **METHOD FOR TREATING ANXIETY IN OLDER SUBJECTS**
VERFAHREN ZUR BEHANDLUNG VON ANGSTSTÖRUNGEN BEI ÄLTEREN PERSONEN
PROCEDE POUR TRAITER L'ANXIETE CHEZ DES SUJETS AGES

(30) Priority: 25.04.2002 US 375462 P
(43) Date of publication of application: 02.02.2005
(73) Proprietor: ELI LILLY AND COMPANY, Indianapolis IN 46285 (US)
(72) Inventor: GERLAI,Robert,Thomas Uni.of Toronto at Mississauga, Mississauga, Ontario, L5L 1C6 (CA)
(74) Representative: Kent, Lindsey Ruth
(86) International application number: PCT/US2003/010473
(87) International publication number: WO 2003/090772

(56) References cited:
- WO-A-01/62801
- US-A1- 2002 009 445
- SEUBERT P ET AL: "ISOLATION AND QUANTIFICATION OF SOLUBLE ALZHEIMER'S BETA-PEPTIDE FROM BIOLOGICAL FLUIDS" NATURE, NATURE PUBLISHING GROUP, LONDON, GB, vol. 359, no. 6393, 24 September 1992 (1992-09-24), pages 325-327, XP000616173 ISSN: 0028-0836
- SOLOMON B ET AL: "DISAGGREGATION OF ALZHEIMER BETA-AMYLOID BY SITE-DIRECTED MAB" PROCEEDINGS OF THE NATIONAL ACADEMY OF SCIENCES OF USA, NATIONAL ACADEMY OF SCIENCE, WASHINGTON, DC, US, vol. 94, April 1997 (1997-04), pages 4109-4112, XP002911322 ISSN: 0027-8424
- BARD F ET AL: "Peripherally administered antibodies against amyloid beta-peptide enter the central nervous system and reduce pathology in a mouse model of Alzheimer disease" NATURE MEDICINE, NATURE PUBLISHING GROUP, NEW YORK, NY, US, vol. 6, no. 8, August 2000 (2000-08), pages 916-919, XP002227088 ISSN: 1078-8956
- OLARIU A. ET AL: "Memory deficits and increased emotionality induced by BETA-amyloid (25-35) are correlated with the reduced acetylcholine release and altered phorbol dibutyrate binding in the hippocampus" JOURNAL OF NEURAL TRANSMISSION, vol. 108, no. 8-9, September 2001 (2001-09), pages 1065-1079, XP002357553
- HARKANY T. ET AL: "BETA-Amyloid (1-42)-Induced Cholinergic Lesions in Rat Nucleus Basalis Bidirectionally Modulate Serotonergic Innervation of the Basal Forebrain and Cerebral Cortex" NEUROBIOLOGY OF DISEASE, vol. 8, no. 4, August 2001 (2001-08), pages 667-678, XP002357554
- ST.GEORGE-HYSLOP, P.H. AND WESTAWAY, D.A.: "Antibody clears senile plaques" NATURE, vol. 400, no. 6740, 8 July 1999 (1999-07-08), pages 116-117, XP002357766

## Description

This invention relates to uses of anti-Aβ antibodies for treating anxiety disorders in elderly subjects.

Millions of older people - indeed, the majority - cope constructively with the physical limitations, cognitive changes, and various losses, such as bereavement, that frequently are associated with later life. The capacity for sound mental health among older adults notwithstanding, a substantial proportion of the population 55 and older - almost 20 percent of this age group - experience specific mental disorders that are not part of "normal" aging. The data below represent the 1-year prevalence (%) of various mental disorders among Americans above age 55. In the same study, the prevalence of any mental disorder was 19.8% and the prevalence of severe cognitive impairment was 6.6%.

| | % | | % |
|---|---|---|---|
| any anxiety disorder | 11.4 | any mood disorder | 4.4 |
| simple phobia | 7.3 | major depressive episode | 3.8 |
| social phobia | 1.0 | unipolar major depression | 3.7 |
| agoraphobia | 4.1 | dysthymia | 1.6 |
| panic disorder | 0.5 | schizophrenia | 0.6 |
| obsessive-compulsive disorder | 1.5 | other | 0.6 |

Information about the course and treatment of anxiety lags behind that of other common mental conditions in the elderly, such as depression and Alzheimer's. Anxiety is at least as common in the old as in the young, although how and when it appears is distinctly different in older adults.

Overall, community-based prevalence estimates indicate that about 11.4 percent of adults aged 55 years and older meet criteria for an anxiety disorder in 1 year. Phobic anxiety disorders are among the most common mental disturbances in late life. Anxiety symptoms that do not fulfill the criteria for specific syndromes are reported in as many as 17 percent of older men and 21 percent of older women.

Drugs used to treat anxiety disorders overlap significantly with those used to treat depression, and include selective serotonin reuptake inhibitors (SSRIs), tricyclic antidepressants, benzodiazepines, beta blockers, and monoamine oxidase inhibitors (MAOIs). Benzodiazepines are marginally effective at best in treating chronic anxiety in older patients. The half-life of certain benzodiazepines and their metabolites may be significantly extended in older patients (particularly for the compounds with long half-life). If taken over extended periods, even short-acting benzodiazepines tend to accumulate in older individuals. Thus, it is generally recommended that any use of benzodiazepines be limited to discrete periods (less than 6 months) and that long-acting compounds be avoided in this population. Side effects of benzodiazepines may include drowsiness, fatigue, psychomotor impairment, memory or other cognitive impairment, confusion, paradoxical reactions, depression, respiratory problems, abuse or dependence problems, and withdrawal reactions. Benzodiazepine toxicity in older patients includes sedation, cerebellar impairment (manifested by ataxia, dysarthria, incoordination, or unsteadiness), cognitive impairment, and psychomotor impairment. Psychomotor impairment from benzodiazepines can have severe consequences, leading to impaired driver skills, motor vehicle crashes, and falls.

Buspirone, an anxiolytic (antianxiety) agent that is chemically and pharmacologically distinct from benzodiazepines may require up to 4 weeks to take effect, and significant adverse reactions to buspirone are found in 20 to 30 percent of anxious older patients, including most frequently, gastrointestinal symptoms, dizziness, headache, sleep disturbance, nausea/vomiting, uneasiness, fatigue, and diarrhea.

Thus, it can be concluded from this brief survey of anxiety in older persons that these are serious and costly conditions for which current pharmaceutical agents provide varying degrees of effectiveness, but often with risky adverse reactions and a real possibility of adverse interactions with the many other agents that older people commonly use for other ailments. There is an increasing need as many populations age for new pharmaceutical agents that are effective in treating anxiety in the elderly, and that are more compatible with the many other medications that the elderly commonly receive for other diseases and conditions.

Alzheimer's disease (AD), a disorder of pivotal importance to older adults, strikes 8 to 15 percent of people over the age of 65. Alzheimer's disease is one of the most feared mental disorders because of its gradual, yet relentless, attack on memory. Memory loss, however, is not the only impairment. Symptoms extend to other cognitive deficits in language, object recognition, and executive functioning.

Behavioral symptoms-such as psychosis, agitation, depression, and wandering are common and impose tremendous strain on caregivers. Of the behavioral symptoms experienced by patients with Alzheimer's disease, depression and anxiety occur most frequently during the early stages, while psychoses occur later. Though behavioral symptoms have received less attention than cognitive symptoms, they have serious ramifications, such as, patient and caregiver distress, premature institutionalization, and significant compromise of the quality of life of patients and their families. Alzheimer's disease, especially its behavioral symptoms, appears to place patients at risk for abuse by caregivers. Forty to fifty percent of Alzheimer's patients have symptoms of depression and the depression accelerates loss of functioning in everyday activities. Depression in Alzheimer's is different from other depressive disorders [Olin, et al., Am. J. Geriatr. Psychiatry 10: 125-128 and 129-141 (2002)]. Even modest reduction in behavioral symptoms can produce substantial improvements in functioning and quality of life.

New therapies are being studied for their ability to ameliorate or modify the significant memory loss that is characteristic of AD. Among them, lowering the levels of the Aβ peptide in the brain has been proposed to ameliorate memory loss and improve cognitive abilities in animal models of AD, and development of pharmaceutical agents to reduce Aβ is in progress. Among the pharmaceutical approaches being studied for ameliorating the effects of Aβ is the use of antibodies that bind Aβ peptide.

Olariu A. *et al* discloses chronically infusing a β-amyloid fragment (25-35) into the brains of laboratory animals and observing an increase in the level of anxiety in the animals (Journal of Neural Transmission, vol. 108, no. 8-9, September 2001, pp. 1065-1079). Harkany T. *et al* discloses that Aβ infusion into the magnocellular nucleus basalis (MBN) of a rat impels excitatory injury of the cholinergic projection neurons and elicits significant anxiety in the elevated plus maze model (Neurobiology of Disease, vol. 8, no. 4, August 2001, pp. 667-678).

No link between the anxiety disorders discussed above and antibodies that bind to an Aβ peptide is known. Consequently, drugs potentially ameliorating Aβ-related conditions and pathologies have not been tested with regard to their effects on behavior, other than those associated with cognitive abilities.

It was surprisingly found that when mice were injected twice within a week before fear conditioning with an antibody that binds to Aβ peptide, they exhibited significantly reduced "long-body" posture, a behavioral trait also called "stretch attend posture" in the literature. This behavior is known to be elicited in rodents by pain or fear (e.g. electric shocks, or stimuli previously associated with the shocks, or stimuli associated with natural predators of rodents). This reduction, observed in elderly (11 months old) wild type and transgenic mice that overproduce Aβ in their brains, represents reduced fear or reduced anxiety, which is likely to also affect mood. Most notably, the effect of anti-Aβ antibody was significant both in transgenic mice and in wild-type mice. In summary, it has been surprisingly discovered that administering an agent that modulates levels of Aβ to aged mice reduces anxiety in the mice, regardless of their status with respect to Aβ.

### BRIEF SUMMARY OF THE INVENTION

Accordingly, the present invention is a use of an anti-Aβ antibody for the manufacture of a medicament for treating an anxiety disorder in an elderly subject of at least 55 years old, wherein said antibody binds to an epitope between amino acids 13 and 28 of human Aβ.

### DETAILED DESCRIPTION OF THE INVENTION

The term "treating" includes prophylaxis (preventing), amelioration (reducing or reversing), or elimination of a sign, symptom, condition, disease, or disorder.

"Anxiety disorder" is a generic term for disorders that involve anxiety. The five major anxiety disorders are panic disorder, obsessive-compulsive disorder, post-traumatic stress disorder, generalized anxiety disorder and phobias (including social phobia, also called social anxiety disorder). Among the anxiety disorders that are treated in the practice of the present invention include are obsessive-compulsive disorder, panic disorder, panic attack, agoraphobia, post-traumatic stress disorder, social phobia, disruptive behavior disorder, and chronic fatigue syndrome.

Most of the disorders discussed here are described and categorized in the DIAGNOSTIC AND STATISTICAL MANUAL OF MENTAL DISORDERS, (4th edition, 1994), published by the American Psychiatric Association (hereinafter referred to as DSM or DSM-IV). In the discussion below, the DSM codes for the disorders will be given where appropriate.

Obsessive-compulsive disorder, DSM 300.3, is characterized by recurrent obsessions or compulsions that are severe enough to be time consuming or cause distress or impairment of the patient's life. Obsessions are persistent ideas, thoughts, impulses or images that are recognized by the patient to be intrusive and inappropriate and cause anxiety or distress. The individual senses that the obsession is alien, not under control and not the kind of thought that the patient would expect to have. Common obsessions include repeated thoughts about contamination, repeated doubts, a need to arrange things in a particular order, aggressive or horrific impulses and sexual imagery. Compulsions are repetitive behaviors, such as hand washing, or mental acts, such as counting or repeating words silently, the goal of which is to prevent or reduce anxiety or distress. By definition, compulsions are either clearly excessive or not realistically connected with that which they are designed to neutralize or prevent.

Panic attack, panic disorder and agoraphobia, categorized as DSM 300.01, 300.21 and 300.22, are characterized by irrational sense of imminent danger or doom, an urge to escape, or a fear of being in a situation from which escape might be difficult. The patient exhibits symptoms such as palpitations, accelerated heart rate, sweating, sensations of shortness of breath, chest pain, nausea, dizziness, fear of dying, and the like, and may have such attacks very frequently.

Social phobia, DSM 300.23, produces a marked and persistent fear of social or performance situations in which embarrassment may occur. Exposure to such a situation may result in a panic attack, or other anxious response. Most often, patients with the disorder simply avoid situations of the type that they dread, producing an obvious dislocation in the patient's life.

Post-traumatic stress disorder, DSM 309.81, afflicts patients following exposure to a traumatic stress involving personal experience of an event involving actual or threatened death of injury. Such traumatic events include experiences such as military combat, personal assault, kidnapping, terrorist attack, torture, natural or man-made disasters, severe accidents, or being diagnosed with a dreaded illness. Learning about such events occurring to others, particularly a family member or close friend, also may produce the disorder. Triggering events that symbolize the traumatic event, such as an anniversary, may recreate the stress and bring on the disorder long after the event is passed. Patients strive to avoid stimuli associated with the trauma, even to the point of amnesia or reduced responsiveness to other people in general.

Diagnosis of these disorders, or the identification of a patient at risk of one or more of them, is to be made by a physician or psychiatrist. It is presently believed that administration of an effective dose of an anti-Aβ agent results in the alleviation of the effects of the disorder from which the patient suffers, or even the elimination of the disorder completely. Diagnosis of anxiety disorders in the elderly may be aided by careful inquiry, as described by Lang, A. J., et al., "Anxiety Disorders: How to Recognize and Treat the Medical Symptoms of Emotional Illness," Geriatrics 56: 24-27, 31-34 (2001). Likewise, diagnosis of depression and anxiety in Alzheimer's patients may be more challenging than in other elderly patients. Recent diagnostic criteria for depression in Alzheimer's disease will aid the diagnosis [Olin, et al., Am. J. Geriatr. Psychiatry 10:125-128 and 129-141 (2002)].

"Anxiety" means the subjective unpleasant feeling of nervousness or distress in response to a feared situation (symptoms), sometimes accompanied by physiological signs including nausea, trembling, breathlessness, sweating and increased heart beat. Mental disorders characterized by felt anxiety or related symptoms are classified as "anxiety Disorders." The ability of an agent to treat anxiety and related disorders may be demonstrated using the techniques described hereinbelow or the well-known fear-potentiated startle and elevated plus maze models of anxiety [e.g., Davis, Psychopharmacology, 62:1 (1979); Lister, Psychopharmacology, 92: 180-185 (1987); and United States patent No. 5,750,566].

"Elderly subject" means a subject older than the average age of menopause for the species and culture (if relevant) of which the subject is a member, assuming adequate nutrition and general health. The average age of menopause for humans in the United States is 51 years of age [The Endocrine Society, 4350 East West Highway, Suite 500, Bethesda, Maryland 20814-4426; www.endo-society.org]. The term "subject" or "patient" for purposes of the present invention is any warm-blooded animal such as, but not limited to, a mouse, guinea pig, dog, horse, or human. Preferably, the subject is a mammal, more preferably rodent or primate, and most preferably, human.

"Administering" is the act of introducing a substance into the body of a subject, and may be achieved by oral, intravenous, intraperitoneal, subcutaneous, intramuscular, or intraparenchimal routes, among others. The antibodies are administered to a subject as identified herein using standard administration techniques, such as by intravenous, intraperitoneal, subcutaneous, pulmonary, transdermal, intramuscular, intranasal, buccal, sublingual, infusion, or suppository administration. The preferred routes of administration are intravenous, subcutaneous, and intraperitoneal. More preferred is either intravenous or subcutaneous.

"Effective dose" means an amount of a substance that leads to measurable and beneficial effects, i.e. significant efficacy. The particular effective amount or dose of compound administered according to this invention will of course be determined by the particular circumstances surrounding the case, including the compound administered, the route of administration, the particular condition being treated, and similar considerations. A typical daily dose will contain from about 0.01 mg/kg to about 100 mg/kg of the active compound of this invention. Preferably, daily doses will be about 0.05 mg/kg to about 50 mg/kg, more preferably from about 0.1 mg/kg to about 25 mg/kg. The frequency of dosing may be daily or once, twice, three times or more per week or per month, as needed to effectively treat the condition.

"Anti-Aβ antibody" means an immunoglobulin molecule (preferably an IgG) that recognizes, binds, and (or) sequesters Aβ peptide.

"Aβ peptide" and "Aβ" refer to a peptide that is derived from amyloid precursor protein (Alzheimer's disease amyloid A4 protein [Precursor], "APP") by proteolytic cleavage. Full-length Aβ peptides are from 39 to 43 amino acids long in humans, for example. Full length Aβ peptide may undergo further cleavage *in vivo* to produce Aβ fragments that are shorter at the N-terminus, at the C-terminus, or both, by one to several amino acids. Full-length Aβ peptide or fragments thereof may be used also as antigens to raise antibodies that bind Aβ peptide. Among the many Aβ peptide fragments used for this purpose, the Aβ 13-28 fragment (conjugated via m-maleimidobenzoyl-N-hydroxysuccinimide ester to an anti-CD3 antibody) was used to raise antibody 266 [Seubert, P. et al., Nature 359:325 - 327 (1992)].

"Conditions associated with Aβ" include clinical or pre-clinical Alzheimer's disease, Down's syndrome, and chronic amyloid angiopathy [Grabowski T. J., et al., Ann. Neuro/. 49: 697-705 (2001); Vinters H. V., Ann. Neurol. 49: 691-3 (2001)]. Regardless of the cause, the diagnosis of AD is made clinically by the finding of progressive memory loss with increasing inability to participate in activities of daily living. Mild cognitive impairment may be associated with pre-clinical Alzheimer's disease.

By "antibody" is meant a whole antibody, including without limitation an animal-derived antibody (e.g., murine), chimeric, humanized, human sequence, recombinant, transgenic, grafted and single chain antibody, and the like, or any fusion proteins, conjugates, fragments, or derivatives thereof. An antibody comprises protein resembling an antibody in the broadest sense in that the protein comprises a binding site for an antigen, which binding site is comprised of three pairs of complementarity determining regions. Antibody includes a whole immunoglobulin molecule, a monoclonal antibody, a chimeric antibody, a humanized antibody; a human antibody, or an immunologically effective fragment of any of these. An antibody fragment, or simply fragment, means an Fv, a disulfide linked Fv, scFv, Fab, Fab', or F(ab')₂ fragment, which terms are well known in the art. In some contexts, herein, fragments will be mentioned specifically for emphasis; nevertheless, it will be understood that regardless of whether fragments are specified, the term "antibody" includes such fragments as well as single-chain forms. As long as a protein retains the ability specifically to bind its intended target, it is included within the term "antibody." Also included within the definition "antibody" are single chain forms. Preferably, but not necessarily, the antibodies useful in the invention are produced recombinantly. Antibodies may or may not be glycosylated, though glycosylated antibodies are preferred under some circumstances, such as when prolonged residence in the body is desirable, or when minimum risk of developing neutralizing antibodies. Antibodies, except perhaps for certain types in which cross-linking between chains is accomplished by peptide or other chemical chains, are properly cross-linked via disulfide bonds.

The basic antibody'structural unit is known to comprise a tetramer. Each tetramer is composed of two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function.

Light chains are classified as kappa and lambda. Heavy chains are classified as gamma, mu, alpha, delta, or epsilon, and define the antibody's isotype as IgG, IgM, IgA, IgD and IgE, respectively. IgG isotypes are preferred. Of the IgG subclasses, IgG1 and IgG4 are preferred.

By "humanized antibody" is meant an antibody that is composed partially or fully of amino acid sequences derived from a human antibody germline by altering the sequence of an antibody having non-human complementarity determining regions (CDR). A humanized immunoglobulin does not encompass a chimeric antibody, having a mouse variable region and a human constant region. However, the variable region of the antibody and even the CDR are humanized by techniques that are by now well known in the art. The framework regions of the variable regions are substituted by the corresponding human framework regions leaving the non-human CDR substantially intact. As mentioned above, it is sufficient for use in the methods of the invention, to employ an immunologically specific fragment of the antibody, including fragments representing single chain forms.

Preparation of antibodies for use in the present invention may be carried out by methods well known in the art, including preparing monoclonal antibodies using well known techniques and screening for high affinity antibodies, or by first identifying a monoclonal antibody having reasonably high affinity and then improving the affinity using well known methods [e.g., US Patent Nos. 5,976,562, 5,824514, 5,817,483, 5,814,476, 5,763,192, 5,723,323; WO97/29131; Huse, W.D., et al., Internat'l Rev. Immunol. 10:129-137 (1993); Yelton, D.E., et al., J. Immunol. 155:1994-2004 (1995); Wu, H., et al., Proc. Natl. Acad. Sci. (USA) 95:6037-6042 (1998); Crameri, A., et al., Nature Medicine 2:100-103 (1996); Stemmer, Proc. Natl. Acad. Sci. (USA) 91:10747-10751 (1994); Stemmer, Nature 370:389-391 (1994)].

The antibodies used in the present invention will most advantageously be expressed in recombinant hosts and purified using well known techniques [Page, M.J. & Sydenham, M.A., Bio/Technol. 9, 64-68 (1991); Carroll, A.R, et al., Mol. Immunol. 29, 821-827 (1992); Coloma, M.J., et al., J. Immunol. Meth. 152, 89-104 (1992); Bebbington, C.R., et al., Bio/Technol. 10, 169-175 (1992); Deyev, S., et al., FEBS Lett. 330, 111-113 (1993); Bender, E., et al., Hum. Antibodies Hybridomas 4, 74-79 (1993); Norderhaug, L., et al., J. Immunol. Meth. 204, 77-87 (1997); Poul, M.A., et al., Eur. J. Immunol. 25, 2005-2009 (1995)].

A preferred antibody for use in the present invention is 266, a humanized form of 266, an antibody that binds to the same epitope on Aβ that 266 binds, any antibody comprised of the CDRs of 266, and any antibody that competitively inhibits the binding of 266 and human Aβ. The skilled reader will know how to determine, from among many possible methods that are well known, whether any particular antibody competitively inhibits the binding of 266 and human Aβ. For example, a comparative ELISA method could be used. Wells of a 96-well ELISA plate (e.g., Nunc-Immuno plate, Cat # 439454, NalgeNunc) are coated with Aβ peptide (1-42 is convenient, but other lengths could be used also), optionally conjugated to a larger protein such as albumin. After washing the wells, they are blocked as appropriate, and then rinsed and dried appropriately. A mixture ofbiotinylated 266 antibody (e.g., mouse or humanized; at 0.3 µg/ml final concentration, for example) and a competitor antibody (starting at 750 µg/ml final concentration and serial 3-fold dilutions) are added in a final volume of 100 µl per well. No-competitor and background controls are run. The ELISA plate is incubated at an appropriate temperature for an appropriate length of time, and then the wells are washed. After washing the wells, HRP-conjugated streptavidin (Cat # 21124, Pierce), or equivalent, is added to each well (e.g., 100 µl of 1 µg/ml). The plate is incubated at room temperature for 30 min and washed. For color development, 100 µl/well of ABTS Peroxidase Substrate (Kirkegaard & Perry Laboratories), or equivalent, is added. Color development is stopped and absorbance is read (e.g., at 415 nm). The absorbances are plotted against the log of the competitor concentration, curves are fitted to the data points (e.g., using Prism or equivalent) and the IC50 determined using methods well known in the art. An antibody having an IC50 within about 100-fold of that of 266 is considered to competitively inhibit its binding.

Antibody 266 has the following amino acid sequences as CDRs:

In humanized versions of 266, human framework regions may optionally have substitutions of one to several residues from mouse 266 for the purpose of maintaining the strength or specificity of the binding of humanized antibody 266 [see, Holtzman, et al., WO01/ 62801]. A preferred light chain variable region of a humanized 266 antibody for use in the present invention has the following amino acid sequence: wherein:
Xaa at position 2 is Val or Ile;
Xaa at position 7 is Ser or Thr;
Xaa at position 14 is Thr or Ser;
Xaa at position 15 is Leu or Pro;
Xaa at position 30 is Ile or Val;
Xaa at position 50 is Arg, Gln, or Lys;
Xaa at position 88 is Val or Leu;
Xaa at position 105 is Gln or Gly;
Xaa at position 108 is Lys or Arg; and
Xaa at position 109 is Val or Leu.

A preferred heavy chain variable region of a humanized 266 antibody for use in the present invention has the following amino acid sequence: wherein:
Xaa at position 1 is Glu or Gln;
Xaa at position 7 is Ser or Leu;
Xaa at position 46 is Glu, Val, Asp, or Ser;
Xaa at position 63 is Thr or Ser;
Xaa at position 75 is Ala, Ser, Val, or Thr;
Xaa at position 76 is Lys or Arg;
Xaa at position 89 is Glu or Asp; and
Xaa at position 107 is Leu or Thr.

A particularly preferred light chain variable region of a humanized 266 antibody for use in the present invention has the following amino acid sequence:

A particularly preferred heavy chain variable region of a humanized 266 antibody for use in the present invention has the following amino acid sequence:

A preferred light chain for a humanized 266 antibody for use in the present invention has the amino acid sequence:

A preferred heavy chain for a humanized 266 antibody for use in the present invention has the amino acid sequence:

Another preferred antibody for use in the present invention is an analog of 266, in which an N-glycosylation site within CDR2 of the heavy chain (SEQ ID NO:5) is engineered so as not to be glycosylated. Such an analog comprises a light chain and a heavy chain, wherein the light chain comprises the three light chain complementarity determining regions (CDRs) from mouse monoclonal antibody 266 (SEQ ID NO:1-3), and wherein the heavy chain comprises heavy chain CDR1 and CDR3 from mouse monoclonal antibody 266 (SEQ ID NO: 4 and 6, respectively), and a heavy chain CDR2 having the sequence given by SEQ ID NO: 13: wherein,
Xaa at position 7 is any amino acid, provided that if Xaa at position 8 is neither Asp nor Pro and Xaa at position 9 is Ser or Thr, then Xaa at position 7 is not Asn;
Xaa at position 8 is any amino acid, provided that if Xaa at position 7 is Asn and Xaa at position 9 is Ser or Thr, then Xaa at position 8 is Asp or Pro; and
Xaa at position 9 is any amino acid, provided that if Xaa at position 7 is Asn and Xaa at position 8 is neither Asp nor Pro, then Xaa at position 9 is neither Ser nor Thr.

By "any amino acid" is meant any naturally occurring amino acid. Preferred naturally-occurring amino acids are Ala, Cys, Asp, Glu, Phe, Gly, His, Ile, Lys, Leu, Met, Asn, Pro, Gln, Arg, Ser, Thr, Val, Trp, and Tyr.

A preferred group of antibodies are those having as light chain CDR1-CDR3 the sequences SEQ ID NO: 1 -3, respectively, as heavy chain CDR1 and CDR3 the sequences SEQ ID NO:4 and 6, respectively, and wherein the sequence of heavy chain CDR2 is SEQ ID NO: 13, wherein:
Xaa at position 7 of SEQ ID NO:13 is selected from the group consisting of Ala, Cys, Asp, Glu, Phe, Gly, His, Ile, Lys, Leu, Met, Asn, Pro, Gln, Arg, Ser, Thr, Val, Trp, and Tyr, provided that if Xaa at position 8 is neither Asp nor Pro and Xaa at position 9 is Ser or Thr, then Xaa at position 7 is not Asn;
Xaa at position 8 of SEQ ID NO: 13 is selected from the group consisting of Ala, Cys, Asp, Glu, Phe, Gly, His, Ile, Lys, Leu, Met, Asn, Pro, Gln,-Arg, Ser, Thr, Val, Trp, and Tyr, provided that if Xaa at position 7 is Asn and Xaa at position 9 is Ser or Thr, then Xaa at position 8 is Asp or Pro; and
Xaa at position 9 of SEQ ID NO:13. is selected from the group consisting of Ala, Cys, Asp, Glu, Phe, Gly, His, Ile, Lys, Leu, Met, Asn, Pro, Gln, Arg, Ser, Thr, Val, Trp, and Tyr, provided that if Xaa at position 7 is Asn and Xaa at position 8 is neither Asp nor Pro, then Xaa at position 9 is neither Ser nor Thr.

Another description of the preferred group is: antibodies or fragments thereof having as light chain CDR1-CDR3 the sequences SEQ ID NO:1-3, respectively, as heavy chain CDR1 and CDR3 the sequences SEQ ID NO:4 and 6, respectively, and wherein the sequence of heavy chain CDR2 is selected from the group consisting of:
1) SEQ ID NO:14 wherein:
   Xaa at position 7 of SEQ ID NO: 14 is selected from the group consisting of Ala, Cys, Asp, Glu, Phe, Gly, His, Ile, Lys, Leu, Met, Pro, Gln, Arg, Ser, Thr, Val, Trp, and Tyr;
   Baa at position 8 of SEQ ID NO:14 is selected from the group consisting of Ala, Cys, Asp, Glu, Phe, Gly, His, Ile, Lys, Leu, Met, Asn, Pro, Gln, Arg, Ser, Thr, Val, Trp, and Tyr; and
   Xaa at position 9 of SEQ ID NO: 14 is selected from the group consisting of Ala, Cys, Asp, Glu, Phe, Gly, His, Ile, Lys, Leu, Met, Asn, Pro, Gln, Arg, Ser, Thr, Val, Trp, and Tyr;
2) SEQ1DNO:15 wherein:
   Xaa at position 7 of SEQ ID NO: 15 is Asn;
   Xaa at position 8 of SEQ ID NO:15 is selected from the group consisting of Ala, Cys, Asp, Glu, Phe, Gly, His, Ile, Lys, Leu, Met, Asn, Pro, Gln, Arg, Ser, Thr, Val, Trp, and Tyr; and
   Xaa at position 9 of SEQ ID NO:15 is selected from the group consisting of Ala, Cys, Asp, Glu, Phe, Gly, His, Ile, Lys, Leu, Met, Asn, Pro, Gln, Arg, Val, Trp, and Tyr; and
3) SEQ ID NO:16 wherein:
   Xaa at position 7 of SEQ ID NO: 16 is Asn;
   Xaa at position 8 of SEQ ID NO:16 is selected from the group consisting of Asp and Pro; and
   Xaa at position 9 of SEQ ID NO: 16 is selected from the group consisting of Ser and Thr.

Preferred sequences for CDR2 of the heavy chain include those in which only a single amino acid is changed, those in which only two amino acids are changed, or all three are changed. It is preferred to replace Asn at position 7, or to replace Thr at position 9, or to replace both. Conservative substitutions at one, two, or all three positions are preferred. The most preferred species are those in which Asn at position 7 is replaced with Ser or Thr.

Preferred deglycosylated 266 antibodies for use in the present invention are those in which in CDR2 of the heavy chain (i.e., within SEQ ID NO: 13, as described above):
Xaa at position 7 is selected from the group consisting of Ala, Gly, His, Asn, Gln, Ser, and Thr, provided that if Xaa at position 9 is Ser or Thr, then Xaa at position 7 is not Asn;
Xaa at position 8 is selected from the group consisting of Ala, Gly, His, Asn, Gln, Ser, and Thr; and
Xaa at position 9 is selected from the group consisting of Ala, Gly, His, Asn, Gln, Ser, and Thr, provided that if Xaa at position 7 is Asn, then Xaa at position 9 is neither Ser nor Thr.

An alternate description of preferred declycogsylated 266 antibodies is: antibodies or fragments thereof having as light chain CDR1-CDR3 the sequences SEQ ID NO: 1-3, respectively, as heavy chain CDR1 and CDR3 the sequences SEQ ID NO:4 and 6, respectively, and wherein the sequence of heavy chain CDR2 is selected from the group consisting of:
1) SEQ ID NO:17 wherein:
   Xaa at position 7 of SEQ ID NO: 17 is selected from the group consisting of Ala, Gly, His, Gln, Ser, and Thr;
   Xaa at position 8 of SEQ ID NO: 17 is selected from the group consisting of Ala, Gly, His, Asn, Gln, Ser, and Thr; and
   Xaa at position 9 of SEQ ID NO: 17 is selected from the group consisting of Ala, Gly, His, Asn, Gln, Ser, and Thr; and
2) SEQ ID NO:18 wherein:
   Baa at position 7 of SEQ ID NO:18 is Asn;
   Xaa at position 8 of SEQ ID NO: 18 is selected from the group consisting of Ala, Gly, His, Asn, Gln, Ser, and Thr; and
   Xaa at position 9 of SEQ ID NO:18 is selected from the group consisting of Ala, Gly, His, Asn, and Gln.

A preferred humanized antibody for use in the present invention has the light chain variable region of SEQ ID NO:7 and a heavy chain variable region of SEQ ID NO:19 wherein:
Xaa at position 1 is Glu or Gln;
Xaa at position 7 is Ser or Leu;
Xaa at position 46 is Glu, Val, Asp, or Ser;
Xaa at position 56 is any amino acid, provided that if Xaa at position 57 is neither Asp nor Pro and Xaa at position 59 is Ser or Thr, then Xaa at position 56 is not Asn;
Xaa at position 57 is any amino acid, provided that if Xaa at position 56 is Asn and Xaa at position 58 is Ser or Thr, then Xaa at position 57 is Asp or Pro; and
Xaa at position 58 is any amino acid, provided that if Xaa at position 56 is Asn and Xaa at position 57 is neither Asp nor Pro, then Xaa at position 58 is neither Ser nor Thr
Xaa at position 63 is Thr or Ser;
Xaa at position 75 is Ala, Ser, Val, or Thr;
Xaa at position 76 is Lys or Arg;
Xaa at position 89 is Glu or Asp; and
Xaa at position 107 is Leu or Thr.

A preferred humanized antibody for use in the present invention has the light chain variable region of SEQ ID NO:9 and a heavy chain variable region of SEQ ID NO:20: wherein:
Xaa at position 56 is any amino acid, provided that if Xaa at position 57 is neither Asp nor Pro and Xaa at position 59 is Ser or Thr, then Xaa at position 56 is not Asn;
Xaa at position 57 is any amino acid, provided that if Xaa at position 56 is Asn and Xaa at position 58 is Ser or Thr, then Xaa at position 57 is Asp or Pro; and
Xaa at position 58 is any amino acid, provided that if Xaa at position 56 is Asn and Xaa at position 57 is neither Asp nor Pro, then Xaa at position 58 is neither Ser nor Thr.

A preferred humanized antibody for use in the present invention has the light chain variable region of SEQ ID NO:11 and a heavy chain given by SEQ ID NO:21: wherein:
Xaa at position 56 is any amino acid, provided that if Xaa at position 57 is neither Asp nor Pro and Xaa at position 59 is Ser or Thr, then Xaa at position 56 is not Asn;
Xaa at position 57 is any amino acid, provided that if Xaa at position 56 is Asn and Xaa at position 58 is Ser or Thr, then Xaa at position 57 is Asp or Pro; and
Xaa at position 58 is any amino acid, provided that if Xaa at position 56 is Asn and Xaa at position 57 is neither Asp nor Pro, then Xaa at position 58 is neither Ser nor Thr.

Preferred deglycosylated 266 antibodies having the heavy variable region according to SEQ ID NO: 19, SEQ ID NO:20, and SEQ ID NO:21 are those wherein:
Xaa at position 56 is selected from the group consisting of Ala, Gly, His, Asn, Gln, Ser, and Thr, provided that if Xaa at position 58 is Ser or Thr, then Xaa at position 56 is not Asn;
Xaa at position 57 is selected from the group consisting of Ala, Gly, His, Asn, Gln, Ser, and Thr; and
Xaa at position 58 is selected from the group consisting of Ala, Gly, His, Asn, Gln, Ser, and Thr, provided that if Xaa at position 56 is Asn, then Xaa at position 58 is neither Ser nor Thr.

Preferred sequences for CDR2 (positions 56, 57, and 58) of the heavy chain SEQ ID NO: 19, SEQ ID NO:20, and SEQ ID NO:21 include those in which only a single amino acid is changed, those in which only two amino acids are changed, or all three are changed. It is preferred to replace Asn at position 56. It is preferred to replace Thr at position 58 with an amino acid other than Ser. It is preferred to not destroy the N-glycosylation site in the CDR2 of the 266 heavy chain by replacing Ser at position 57 with Pro or Asp. Conservative substitutions at one, two, or all three positions are preferred. The most preferred species are those in which Asn at position 56 is replaced with Ser or Thr. Particularly preferred antibodies are those in which Ser or Thr is at position 56, Ser is at position 57, and Thr is at position 58 of SEQ ID NO: 19, SEQ ID NO:20, or SEQ ID NO:2 1.

The most preferred species are antibodies comprising a light chain of SEQ ID NO:11 and a heavy chain of SEQ ID NO:21, wherein in SEQ ID NO:2 1, Xaa at position 56 is Ser, Xaa at position 57 is Ser, and Xaa at position 58 is Thr ("N56S"), or wherein in SEQ ID NO:21, Xaa at position 56 is Thr, Xaa at position 57 is Ser, and Xaa at position 58 is Thr ("N56T").

The preparation of an acceptable pharmaceutical preparation of the antibodies used in the present invention, including its strength, excipients, pH, isotonicity, presentation, dosage form, and the like, is well known to the skilled person. Pharmaceutical compositions for use in the present invention should be appropriate for the selected mode of administration, and pharmaceutically acceptable excipients such as, buffers, surfactants, preservatives, solubilizing agents, isotonicity agents, stabilizing agents and the like are used as appropriate. Remington's Pharmaceutical Sciences, Mack Publishing Co., Easton PA, latest edition, incorporated herein by reference, provides a compendium of formulation techniques as are generally known to practitioners. Pharmaceutical preparations for use in the present invention should be sterile or at least nearly so, and if necessary preserved or rendered bacteriostatic.

The following example(s) are intended to illustrate, not limit, the invention.

### Example 1

Adult, 11 month old females, wild type control and homozygous PDAPP transgenic mice originating from a hybrid genetic background (DBA - C57BL/6 - Swiss Webster) [Games et al., Nature. 373:523-527 (1995)], were tested. Approximately 50% of the mice from each genotype group (for sample sizes see tables herein) received 500 µg of mouse monoclonal antibody 266.2 and the other 50% of the mice received phosphate buffered saline (PBS) vehicle administered intra-peritoneally 9 days and 2 days prior to start of behavioral experiments. The behavioral tests were conducted in a fully randomized and blind manner, i.e. the experimenter had no knowledge of the genotype or the drug treatment history of the subjects. Furthermore, mice were tested in four test chambers so that at any given time one mouse was being tested from each of the four (2 genotypes x 2 injection groups) groups. This way any potential circadian changes must have affected all groups in an identical manner.

Prior to testing, and between testing days, animals were group housed (4 mice per cage) in standard plastic cages (32.5 x 15 x 15 cm, length x width x depth) with sawdust bedding, and maintained on a 12/12 hr light/dark cycle (lights on at 6 a.m.) with constant temperature (21 C°) and 45% relative humidity. Food and water were available *ad libitum.*

Four behavioral sessions (6 min each) were conducted over a four-day period as described in detail [Fitch, et al., Hippocampus, 12: 4-17 (2002)]. Briefly, on day one, a habituation session in a neutral context was conducted (behavior was not quantified). This was followed by a training paradigm on day two. Subsequently, on day three, animals were tested in the context memory test and then finally, the cue memory test was conducted on day four. Following each trial, animals were returned to their home cage. Transgenic and wild type mice were randomly assigned to each of four fear conditioning chambers so that 2 transgenic (one treated with the antibody and the other with vehicle) and two wild type mice (one treated with the antibody and the other with vehicle) were running concurrently exactly at the same time. Order of testing was maintained throughout the tests. Each animal was tested in the same chamber in which it was trained. Behavioral experimentation and quantification of data were done blind.

In the neutral context, all animals were exposed to a 'safe' environment in which no shocks or tone cues were delivered. This environment was the basic test chamber but visual and tactile contextual cues of the chamber were altered by replacing the shock grid with a perforated acrylic sheet and by installing wall inserts (yellow cartoon paper) inside the cage. Fresh bedding was placed in the drop pan underneath the floor cover of the chamber to provide a familiar (home cage) smell. Between mice the chamber was cleaned with Petzyme (Petsmart, Pacific Coast Distributing, Phoenix, AZ). This same 'neutral context' was later used for testing tone cue responses. After having been exposed to the neutral context, mice were placed into the test chamber designated 'unsafe' where the wall inserts and acrylic floor were removed. Bedding was removed from the drop pan and the chamber and drop pan were cleaned with Steris (St. Louis, MO) 'Coverage Spray' disinfectant before each subject trial. By using the neutral and unsafe contexts we hoped to facilitate discrimination of relevant contextual cues from other cues, e.g. human handling or cues of the test room, which were always present and which should not be associated with the shock. In the unsafe context (training) subjects were given 10 - 15 s to acclimatize before behavioral recording was begun. For the first 160 s of the training no stimulation was administered. This adaptation period was followed by a 20 s tone cue (80 db, 3000 Hz), which was co-terminated with a 1 sec 0.7 mA scrambled electric foot shock: This stimulus presentation was repeated at 220 s and again at 280 s for a total of three tone/shock pairings during the 360 s long training session.

The following day, contextual-cues-elicited fear responses were recorded for a 360 s period in the chamber in which training was previously conducted (unsafe context). No tone or shock was presented during this context test. Between individual subject trials, chambers were cleaned with 'Petzyme', an agent that provided a novel odor cue. The change in cleaning agents was made in order to minimize the possibility of mice using a salient olfactory cue to identify the unsafe context and thus exhibit their response based on elemental rather than contextual information.

On the final day of the paradigm, elemental tone cue-associated learning was tested in the safe neutral chamber with wall inserts and acrylic floor cover present, and with clean bedding in the drop pan beneath the floor. During this cue test, animals were presented with tone cues identical in amplitude, frequency, and timing of delivery to those given during training, however, no shock was administered. Following each training or test trial, animals were returned to their home cages.

Behavior of the mice was video-recorded with Camcorders (Sony, DCR TRV-20 mini DVCam) and later replayed on a digital VCR (Sony DVCAM DSR-20 digital VCR). Quantification of behavior was conducted using Observer Video Pro software (Noldus, Vageningen, The Netherlands). The software allows the experimenter to quantify predefined motor and posture patterns. The software also makes it possible for the experimenter to control the digital VCR and to synchronize the computer's internal clock with the time stamp generated by the VCR. The following behavioral parameter is quantified: long-body (also known in the literature as "stretch attend posture"), hind paws are anchored while the front of the body is moving forward, body is elongated (stretched) and is kept very close to the ground. For the training session the relative duration of long-body was calculated for two intervals, the period preceding the first shock (0-179 sec, neutral acclimation period) and the period including and following the first shock (179 - 360 sec, period during which subjects experience pain due to electric shock). For the context test, data are expressed for a single interval, the entire period (.0- 360 sec, period during which subjects experience fear due to presence of contextual stimuli). For the cue test, data are expressed again for two intervals, for the period preceding the first tone cue (0-160 sec, period during which no signs of danger are present) and for the period following this (160 - 360 sec, a period during which tone cues previously associated with the shock are delivered).

Statistical analyses were conducted using SYSTAT 10 statistical software package on a Compaq PC. Three-way or two-way repeated measures analysis of variance (ANOVA) was conducted to test the effects of genotype (wild type or transgenic), the effects of injection (antibody or vehicle control) and interval, which is the repeated measure factor.

For the training session, significant genotype, antibody, and interval effects were found (Table 1). The interaction terms interval x genotype and interval x genotype x antibody were also significant.

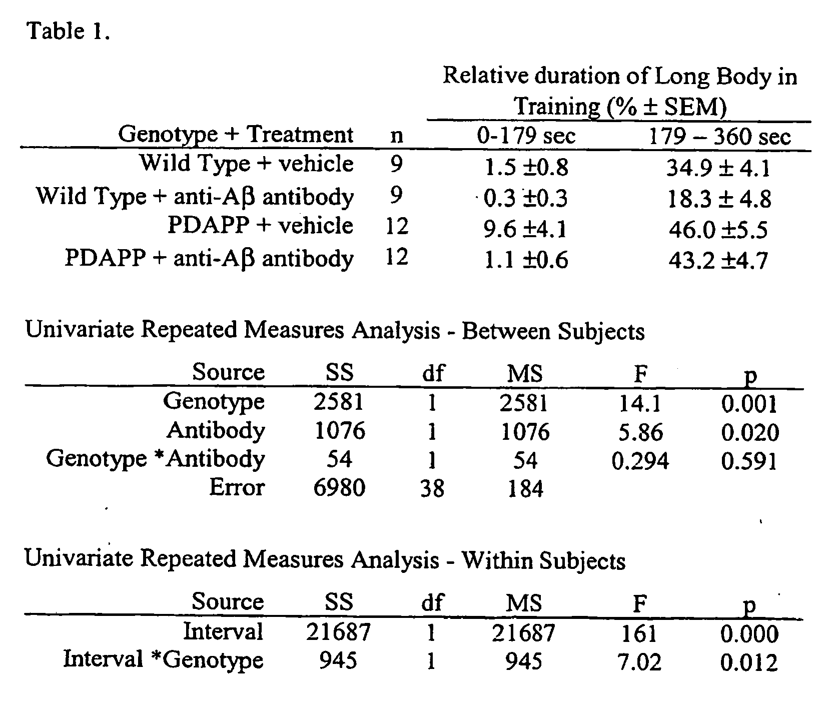

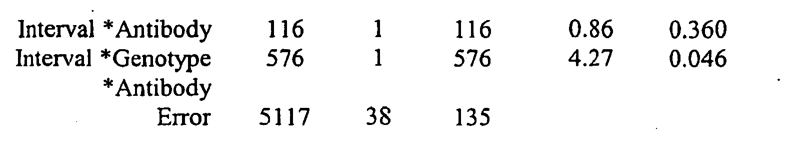

Briefly, long-body posture was almost entirely absent before electric shocks were administered, confirming that under baseline condition, i.e. without the presence of pain or fear, mice do not exhibit this behavior. Interestingly, PDAPP transgenic mice did show some appreciable level of long-body posture even during the first period of training when no shocks were given, but this was significantly reduced by the antibody treatment. In response to electric shocks a significant increase of long-body posture is observed in all mice, but this increase was somewhat smaller in wild type animals compared to PDAPP transgenic mice. Furthermore, antibody treated mice, especially the wild type mice, showed a blunted increase of long-body posture.

Similar to the training session, significant antibody effects were observed in the context test and the effect of transgene was also significant (Table 2).

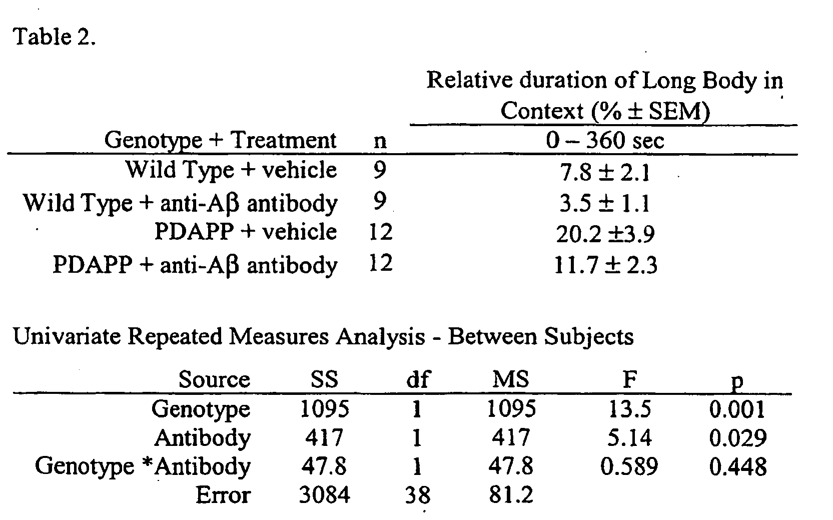

The antibody reduced long-body posture in the PDAPP mice almost to the level of the vehicle injected wild type mice. But it is also notable that the antibody had a similar long-body posture reducing effect even when injected in the wild type mice as compared to vehicle injected wild type mice. Variance analysis showed that indeed the effect of the transgene and the effect of the antibody injection did not interact with each other (Table 2, transgene x antibody interaction is non-significant), i.e. the presence of the transgene increased, while antibody injection decreased the amount of long-body posture exhibited.

In the cue test a similar pattern of results was seen but the effects were not statistically significant (Table 3), most probably due to the fact that in this test long-body posture was hardly seen. Nevertheless, the effect of antibody treatment was close to significance (p = 0.065).

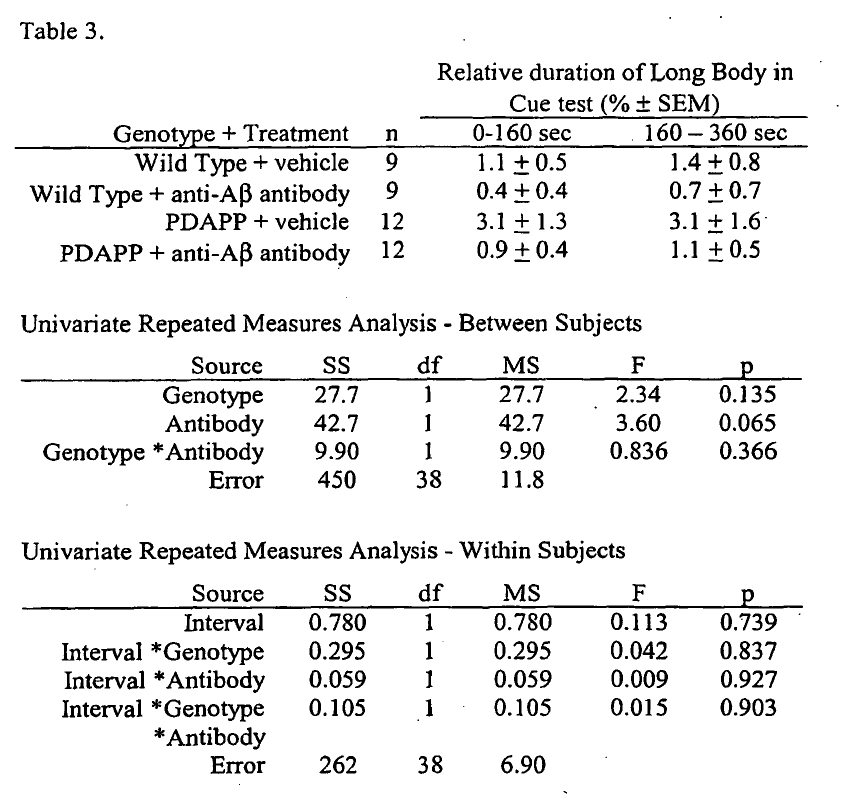

The fear conditioning paradigm allowed us to investigate shock, contextual stimuli, or tone cue induced behavioral responses. Furthermore, software aided event recording made it possible for us to quantify a posture pattern, long-body, which is associated with fear. This behavior showed a consistent increase in PDAPP mice as compared to wild type control. This increase may be due to Aβ deposition or overexpression of the mutant form of APP in the transgenic mice. Consistent with this, but also surprisingly, our analysis also revealed a significant long-body posture reducing effect of the anti-Aβ antibody treatment.

Note the elevated amount of long-body posture exhibited in response to the three shock-tone pairing during the second half of the training session (3-6 min). This behavior is believed to be the expression of a mild level of anxiety. Also note the consistent reduction of this behavior by antibody treatment in both genotype groups.

Long-body posture has been observed under aversive conditions in mice when cues associated with natural predators are present [Blanchard, *et al.,* Risk assessment and animal models of anxiety. In: Olivier et al. (eds.) Animal models in psychopharmacology. Advances in pharmacological sciences. Basel: Birkhauser Verlag. pp. 117-134 (1991)]. This behavior is also evoked in mice by other fear inducing stimuli including electric shocks or the context in which the shocks were delivered [Fitch, *et al.* 2002; Gerlai, et al., J. Neuroscience 19:9538-9549 (1999)]. Thus, long-body has been interpreted as a sign of fear [Blanchard, *et al.* (1991); Fitch, *et al.* (2002); Gerlai, *et al.* (1999)]. Accordingly, increased long-body posture may in fact represent increased fear in PDAPP mice compared to wild type control, and decreased long-body posture elicited by the injection of anti-Aβ antibody may represent reduction of fear.

It is important to emphasize that the decrease of long-body posture was observed both in PDAPP mice and also in wild type control. This suggests that perhaps Aβ lowering may have a beneficial, i.e. anxiolytic, effects not only in AD patients but perhaps in the non-demented elderly population as well. Our present results thus raise a new and unexpected possibility: anti-Aβ agents may represent a novel therapeutic application in the treatment of anxiety and related disorders.

## Claims

1. Use of an anti-Aβ antibody for the manufacture of a medicament for treating an anxiety disorder in an elderly human subject of at least 55 years old, wherein said antibody binds to an epitope between amino acids 13 and 28 of human Aβ.

2. The use of Claim 1, wherein said anxiety disorder is selected from the group consisting of obsessive-compulsive disorder, panic disorder, panic attack, agoraphobia, post-traumatic stress disorder, social phobia, disruptive behaviour disorder and chronic fatigue syndrome.

3. The use of Claim 1, wherein the elderly subject is diagnosed with a condition related to Aβ, selected from clinical or preclinical Alzheimer's disease, chronic amyloid angiopathy and Down's syndrome.

4. The use of Claim 1, wherein the elderly subject is not diagnosed with a condition related to Aβ, selected from clinical or preclinical Alzheimer's disease, chronic amyloid angiopathy and Down's syndrome.

5. The use of any one of Claims 1-4, wherein the elderly subject is a human whose age is at least 60 years, 65, years, 70 years, 75 years, 80 years, 85, years, 90 years, 95 years, 100 years, 105 years, or 110 years.

6. The use of any one of Claims 1-5, wherein the anti-Aβ antibody is a human or humanized antibody.

## Patentansprüche

1. Verwendung eines Anti-Aß-Antikörpers zur Herstellung eines Arzneimittels für die Behandlung einer Angststörung bei einem älteren humanen Subjekt mit einem Alter von wenigstens 55 Jahren, worin dieser humane Antikörper an ein Epitop zwischen den Aminosäuren 13 und 28 von humanem Aβ bindet.

2. Verwendung nach Anspruch 1, worin die Angststörung ausgewählt ist aus der Gruppe, die besteht aus obsessiver-kompulsiver Störung, Panikstörung, Panikattacke, Agoraphobie, posttraumatischer Stressstörung, Sozialphobie, disruptiver Verhaltensstörung und chronischem Ermüdungssyndrom.

3. Verwendung nach Anspruch 1, worin der ältere Mensch mit einem mit Aβ zusammenhängenden Zustand diagnostiziert ist, der ausgewählt ist aus einer klinischen oder präklinischen Alzheimer-Krankheit, einer chronischen Amyloidangiopathie und einem Down-Syndrom.

4. Verwendung nach Anspruch 1, worin der ältere Mensch mit einem mit Aβ zusammenhängenden Zustand nicht diagnostiziert ist, der ausgewählt ist aus einer klinischen oder präklinischen Alzheimer-Krankheit, einer chronischen Amyloidangiopathie und einem Down-Syndrom.

5. Verwendung nach einem der Ansprüche 1 bis 4, worin das ältere Subjekt ein Mensch mit einem Alter von wenigstens 60 Jahren, 65 Jahren, 70 Jahren, 75 Jahren, 80 Jahren, 85 Jahren, 90 Jahren, 95 Jahren, 100 Jahren, 105 Jahren oder 110 Jahren ist.

6. Verwendung nach einem der Ansprüche 1 bis 5, worin der Anti-Aß-Antikörper ein humaner oder humanisierter Antikörper ist.

## Revendications

1. Utilisation d'un anticorps anti-Aβ pour la fabrication d'un médicament destiné au traitement d'un trouble de l'anxiété chez un sujet humain âgé, ayant au moins 55 ans, dans laquelle ledit anticorps humain se lie à un épitope entre les acides aminés 13 et 28 d'un Aβ humain.

2. Utilisation selon la revendication 1, dans laquelle ledit trouble de l'anxiété est choisi dans le groupe constitué par le trouble obsessionnel compulsif, le trouble panique, la crise de panique, l'agoraphobie, le syndrome de stress post-traumatique, la phobie sociale, le trouble du comportement perturbateur et le syndrome de fatigue chronique.

3. Utilisation selon la revendication 1, dans laquelle on diagnostique chez le sujet âgé une pathologie en relation avec Aβ, choisie parmi la maladie d'Alzheimer au stade clinique ou préclinique, l'angiopathie amyloïde chronique et le syndrome de Down.

4. Utilisation selon la revendication 1, dans laquelle on ne diagnostique pas chez le sujet âgé une pathologie en relation avec Aβ, choisie parmi la maladie d'Alzheimer au stade clinique ou préclinique, l'angiopathie amyloïde chronique et le syndrome de Down.

5. Utilisation selon l'une quelconque des revendications 1 à 4, dans laquelle le sujet âgé est un humain âgé d'au moins 60 ans, 65 ans, 70 ans, 75 ans, 80 ans, 85, ans, 90 ans, 95 ans, 100 ans, 105 ans ou 110 ans.

6. Utilisation selon l'une quelconque des revendications 1 à 5, dans laquelle l'anticorps anti-Aβ est un anticorps humain ou humanisé.


### SEQUENCE LISTING

<110> Eli Lilly and company
<120> METHOD FOR TREATING ANXIETY AND MOOD DISORDERS IN OLDER SUBJECTS
<130> x15667
<140> 60/375462
   <141> 2002-04-25
<160> 21
<170> PatentIn version 3.1
<210> 1
   <211> 16
   <212> PRT
   <213> Mus sp.
<400> 1
   Arg Ser Ser Gln Ser Leu Ile Tyr Ser Asp Gly Asn Ala Tyr Leu His
   1               5                   10                  15
<210> 2
   <211> 7
   <212> PRT
   <213> Mus sp.
<400> 2
   Lys Val Ser Asn Arg Phe Ser
   1               5
<210> 3
   <211> 9
   <212> PRT
   <213> Mus sp.
<400> 3
   Ser Gln Ser Thr His Val Pro Trp Thr
   1               5
<210> 4
   <211> 5
   <212> PRT
   <213> Mus sp.
<400> 4
   Arg Tyr Ser Met Ser
   1               5
<210> 5
   <211> 17
   <212> PRT
   <213> Mus sp.
<400> 5
   Gln Ile Asn Ser Val Gly Asn Ser Thr Tyr Tyr Pro Asp Thr Val Lys
   1               5                   10                  15
   Gly
<210> 6
   <211> 3
   <212> PRT
   <213> Mus sp.
<400> 6
   Gly Asp Tyr
   1
<210> 7
   <211> 113
   <212> PRT
   <213> Artificial Sequence
<220>
   <223> synthetic construct - humanized
<220>
   <221> MISC_FEATURE
   <222> (2)..(2)
   <223> Xaa=Val or Ile
<220>
   <221> MISC_FEATURE
   <222> (7)..(7)
   <223> Xaa=Ser or Thr
<220>
   <221> MISC_FEATURE
   <222> (14)..(14)
   <223> Xaa=Thr or Ser
<220>
   <221> MISC_FEATURE
   <222> (15)..(15)
   <223> Xaa=Leu or Pro
<220>
   <221> MISC_FEATURE
   <222> (30)..(30)
   <223> Xaa=Ile or Val
<220>
   <221> MISC_FEATURE
   <222> (50)..(50)
   <223> Xaa=Arg, Gln, or Lys
<220>
   <221> MISC_FEATURE
   <222> (88)..(88)
   <223> Xaa=Val or Leu
<220>
   <221> MISC_FEATURE
   <222> (105)..(105)
   <223> Xaa=Gln or Gly
<220>
   <221> MISC_FEATURE
   <222> (108)..(108)
   <223> Xaa=Lys or Arg
<220>
   <221> MISC_FEATURE
   <222> (109)..(109)
   <223> Xaa=Val or Leu
<400> 7
   Asp Xaa Val Met Thr Gln Xaa Pro Leu Ser Leu Pro Val Xaa Xaa Gly
   1               5                   10                  15
   Gln Pro Ala Ser Ile Ser Cys Arg Ser Ser Gln Ser Leu Xaa Tyr Ser
               20                  25                  30
   Asp Gly Asn Ala Tyr Leu His Trp Phe Leu Gln Lys Pro Gly Gln Ser
           35                  40                  45
   Pro Xaa Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Xaa Gly Val Tyr Tyr Cys Ser Gln Ser
                   85                  90                  95
   Thr His Val Pro Trp Thr Phe Gly Xaa Gly Thr Xaa Xaa Glu Ile Lys
               100                 105                 110
   Arg
<210> 8
   <211> 112
   <212> PRT
   <213> Artificial Sequence
<220>
   <223> synthetic construct - humanized
<220>
   <221> MISC_FEATURE
   <222> (1)..(1)
   <223> Xaa=Glu or Gln
<220>
   <221> MISC_FEATURE
   <222> (7)..(7)
   <223> Xaa=Ser or Leu
<220>
   <221> MISC_FEATURE
   <222> (46)..(46)
   <223> Xaa= Glu, Val, Asp, or Ser
<220>
   <221> MISC_FEATURE
   <222> (63)..(63)
   <223> Xaa= Thr or Ser
<220>
   <221> MISC_FEATURE
   <222> (75)..(75)
   <223> Xaa=Ala, Ser, Val, or Thr
<220>
   <221> MISC_FEATURE
   <222> (76)..(76)
   <223> Xaa=Lys or Arg
<220>
   <221> MISC_FEATURE
   <222> (89)..(89)
   <223> Xaa=Glu or Asp
<220>
   <221> MISC_FEATURE
   <222> (107)..(107)
   <223> Xaa=Leu or Thr
<400> 8
   Xaa Val Gln Leu Val Glu Xaa Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Ser Met Ser Trp Val Arg Gln Ala Pro Gly Lys Gly Leu Xaa Leu Val
           35                  40                  45
   Ala Gln Ile Asn Ser Val Gly Asn Ser Thr Tyr Tyr Pro Asp Xaa Val
       50                  55                  60
   Lys Gly Arg Phe Thr Ile Ser Arg Asp Asn Xaa Xaa Asn Thr Leu Tyr
   65                  70                  75                  80
   Leu Gln Met Asn Ser Leu Arg Ala Xaa Asp Thr Ala Val Tyr Tyr Cys
                   85                  90                  95
   Ala Ser Gly Asp Tyr Trp Gly Gln Gly Thr Xaa Val Thr Val Ser Ser
               100                 105                 110
<210> 9
   <211> 113
   <212> PRT
   <213> Artificial
<220>
   <223> Synthetic construct - humanized
<400> 9
   Asp Val Val Met Thr Gln Ser Pro Leu Ser Leu Pro Val Thr Leu Gly
   1               5                   10                  15
   Gln Pro Ala Ser Ile Ser Cys Arg Ser Ser Gln Ser Leu Ile Tyr Ser
               20                  25                  30
   Asp Gly Asn Ala Tyr Leu His Trp Phe Leu Gln Lys Pro Gly Gln Ser
           35                  40                  45
   Pro Arg Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ser Gln Ser
                   85                  90                  95
   Thr His Val Pro Trp Thr Phe Gly Gln Gly Thr Lys Val Glu Ile Lys
               100                 105                 110
   Arg
<210> 10
   <211> 112
   <212> PRT
   <213> Artificial
<220>
   <223> Synthetic construct - humanized
<400> 10
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Arg Tyr
   20 25 30
   Ser Met Ser Trp Val Arg Gln Ala Pro Gly Lys Gly Leu Glu Leu Val
   35 40 45
   Ala Gln Ile Asn Ser Val Gly Asn Ser Thr Tyr Tyr Pro Asp Thr Val
   50 55 60
   Lys Gly Arg Phe Thr Ile Ser Arg Asp Asn Ala Lys Asn Thr Leu Tyr
   65 70 75 80
   Leu Gln Met Asn Ser Leu Arg Ala Glu Asp Thr Ala Val Tyr Tyr Cys
   85 90 95
   Ala Ser Gly Asp Tyr Trp Gly Gln Gly Thr Leu Val Thr Val Ser Ser
   100 105 110
<210> 11
   <211> 219
   <212> PRT
   <213> Artificial
<220>
   <223> Synthetic construct - humanized
<400> 11
   Asp Val Val Met Thr Gln Ser Pro Leu Ser Leu Pro Val Thr Leu Gly
   1 5 10 15
   Gln Pro Ala Ser Ile Ser Cys Arg Ser Ser Gln Ser Leu Ile Tyr Ser
   20 25 30
   Asp Gly Asn Ala Tyr Leu His Trp Phe Leu Gln Lys Pro Gly Gln Ser
   35 40 45
   Pro Arg Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Ser Gln Ser
   85 90 95
   Thr His Val Pro Trp Thr Phe Gly Gln Gly Thr Lys Val Glu Ile Lys
   100 105 110
   Arg Thr Val Ala Ala Pro Ser Val Phe Ile Phe Pro Pro Ser Asp Glu
   115 120 125
   Gln Leu Lys Ser Gly Thr Ala Ser Val Val Cys Leu Leu Asn Asn Phe
   130 135 140
   Tyr Pro Arg Glu Ala Lys Val Gln Trp Lys Val Asp Asn Ala Leu Gln
   145 150 155 160
   Ser Gly Asn Ser Gln Glu Ser Val Thr Glu Gln Asp Ser Lys Asp Ser
   165 170 175
   Thr Tyr Ser Leu Ser Ser Thr Leu Thr Leu Ser Lys Ala Asp Tyr Glu
   180 185 190
   Lys His Lys Val Tyr Ala Cys Glu Val Thr His Gln Gly Leu Ser Ser
   195 200 205
   Pro Val Thr Lys Ser Phe Asn Arg Gly Glu Cys
   210 215
<210> 12
   <211> 442
   <212> PRT
   <213> Artificial
<220>
   <223> Synthetic construct - humanized
<400> 12
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Arg Tyr
   20 25 30
   Ser Met Ser Trp Val Arg Gln Ala Pro Gly Lys Gly Leu Glu Leu Val
   35 40 45
   Ala Gln Ile Asn Ser Val Gly Asn Ser Thr Tyr Tyr Pro Asp Thr Val
   50 55 60
   Lys Gly Arg Phe Thr Ile Ser Arg Asp Asn Ala Lys Asn Thr Leu Tyr
   65 70 75 80
   Leu Gln Met Asn Ser Leu Arg Ala Glu Asp Thr Ala Val Tyr Tyr Cys
                   85                  90                  95
   Ala Ser Gly Asp Tyr Trp Gly Gln Gly Thr Leu Val Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Lys Thr His Thr Cys Pro Pro Cys
       210                 215                 220
   Pro Ala Pro Glu Leu Leu Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser His Glu Asp Pro Glu Val Lys Phe Asn Trp
               260                 265                 270
   Tyr Val Asp Gly Val Glu Val His Asn Ala Lys Thr Lys Pro Arg Glu
           275                 280                 285
   Glu Gln Tyr Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Ala Leu Pro Ala Pro Ile Glu Lys Thr Ile Ser Lys Ala Lys Gly
                   325                 330                 335
   Gln Pro Arg Glu Pro Gln Val Tyr Thr Leu Pro Pro Ser Arg Asp Glu
   340 345 350
   Leu Thr Lys Asn Gln Val Ser Leu Thr Cys Leu Val Lys Gly Phe Tyr
   355 360 365
   Pro Ser Asp Ile Ala Val Glu Trp Glu Ser Asn Gly Gln Pro Glu Asn
   370 375 380
   Asn Tyr Lys Thr Thr Pro Pro Val Leu Asp Ser Asp Gly Ser Phe Phe
   385 390 395 400
   Leu Tyr Ser Lys Leu Thr Val Asp Lys Ser Arg Trp Gln Gln Gly Asn
   405 410 415
   Val Phe Ser Cys Ser Val Met His Glu Ala Leu His Asn His Tyr Thr
   420 425 430
   Gln Lys Ser Leu Ser Leu Ser Pro Gly Lys
   435 440
<210> 13
   <211> 17
   <212> PRT
   <213> Mus sp.
<220>
   <221> MISC_FEATURE
   <222> (7)..(7)
   <223> Xaa at position 7 is any amino acid, provided that if Xaa at position 8 is neither Asp nor Pro and Xaa at position 9 is Ser or Thr, then Xaa at position 7 is not Asn
<220>
   <221> MISC_FEATURE
   <222> (8)..(8)
   <223> Xaa at position 8 is any amino acid, provided that if Xaa at position 7 is Asn and Xaa at position 9 is Ser or Thr, then Xaa at position 8 is Asp or Pro
<220>
   <221> MISC_FEATURE
   <222> (9)..(9)
   <223> Xaa at position 9 is any amino acid, provided that if Xaa at position 7 is Asn and Xaa at position 8 is neither Asp nor Pro, then Xaa at position 9 is neither Ser nor Thr
<400> 13
   Gln Ile Asn Ser Val Gly Xaa Xaa Xaa Tyr Tyr Pro Asp Thr Val Lys
   1 5 10 15
   Gly
<210> 14
   <211> 17
   <212> PRT
   <213> Mus sp.
<220>
   <221> MISC_FEATURE
   <222> (7)..(7)
   <223> Xaa at position 7 is selected from the group consisting of Ala, Cys, Asp, Glu, Phe, Gly, His, Ile, Lys, Leu, Met, Pro, Gln, Arg, Ser, Thr, Val, Trp, and Tyr
<220>
   <221> MISC_FEATURE
   <222> (8)..(8)
   <223> Xaa at position 8 is selected from the group consisting of Ala, Cys, Asp, Glu, Phe, Gly, His, Ile, Lys, Leu, Met, Asn, Pro, Gln, Arg, Ser, Thr, Val, Trp, and Tyr
<220>
   <221> MISC_FEATURE
   <222> (9)..(9)
   <223> Xaa at position 9 is selected from the group consisting of Ala, Cys, Asp, Glu, Phe, Gly, His, Ile, Lys, Leu, Met, Asn, Pro, Gln, Arg, Ser, Thr, Val, Trp, and Tyr
<400> 14
   Gln Ile Asn Ser Val Gly Xaa Xaa Xaa Tyr Tyr Pro Asp Thr Val Lys
   1               5                   10                  15
   Gly
<210> 15
   <211> 17
   <212> PRT
   <213> Mus sp.
<220>
   <221> MISC_FEATURE
   <222> (7)..(7)
   <223> Xaa=Asn
<220>
   <221> MISC_FEATURE
   <222> (8)..(8)
   <223> Xaa at position 8 is selected from the group consisting of Ala, Cys, Asp, Glu, Phe, Gly, His, Ile, Lys, Leu, Met, Asn, Pro**,** Gln, Arg, Ser, Thr, Val, Trp, and Tyr.
<220>
   <221> MISC_FEATURE
   <222> (9)..(9)
   <223> Xaa at position 9 is selected from the group consisting of Ala, Cys, Asp, Glu, Phe, Gly, His, Ile, Lys, Leu, Met, Asn, Pro, Gln, Arg, Ser, Thr, Val, Trp, and Tyr
<400> 15
   Gln Ile Asn Ser Val Gly Xaa Xaa Xaa Tyr Tyr Pro Asp Thr Val Lys
   1               5                   10                  15
   Gly
<210> 16
   <211> 17
   <212> PRT
   <213> Mus sp.
<220>
   <221> MISC_FEATURE
   <222> (7)..(7)
   <223> Xaa=Asn
<220>
   <221> MISC_FEATURE
   <222> (8)..(8)
   <223> Xaa at position 8 is selected from the group consisting of Asp and Pro
<220>
   <221> MISC_FEATURE
   <222> (9)..(9)
   <223> Xaa at position 9 is selected from the group consisting of Ser and Thr
<400> 16
   Gln Ile Asn Ser Val Gly Xaa Xaa Xaa Tyr Tyr Pro Asp Thr Val Lys
   1               5                   10                  15
   Gly
<210> 17
   <211> 17
   <212> PRT
   <213> Mus sp.
<220>
   <221> MISC_FEATURE
   <222> (7)..(7)
   <223> Xaa at position 7 is selected from the group consisting of Ala, Gly, His, Gln, Ser, and Thr
<220>
   <221> MISC_FEATURE
   <222> (8)..(8)
   <223> Xaa at position 8 is selected from the group consisting of Ala, Gly, His, Asn, Gln, Ser, and Thr
<220>
   <221> MISC_FEATURE
   <222> (9)..(9)
   <223> Xaa at position 9 is selected from the group consisting of Ala, Gly, His, Asn, Gln, Ser, and Thr
<400> 17
   Gln Ile Asn Ser Val Gly Xaa Xaa Xaa Tyr Tyr Pro Asp Thr Val Lys
   1 5 10 15
   Gly
<210> 18
   <211> 17
   <212> PRT
   <213> Mus sp.
<220>
   <221> MISC_FEATURE
   <222> (7)..(7)
   <223> Xaa at position 7 is Asn
<220>
   <221> MISC_FEATURE
   <222> (8)..(8)
   <223> Xaa at position 8 is selected from the group consisting of Ala, Gly, His, Asn, Gln, Ser, and Thr
<220>
   <221> MISC_FEATURE
   <222> (9)..(9)
   <223> Xaa at position 9 is selected from the group consisting of Ala, Gly, His, Asn, and Gln
<400> 18
   Gln Ile Asn Ser Val Gly Xaa Xaa Xaa Tyr Tyr Pro Asp Thr Val Lys
   1 5 10 15
   Gly
<210> 19
   <211> 112
   <212> PRT
   <213> Artificial
<220>
   <223> synthetic construct - humanized
<220>
   <221> MISC_FEATURE
   <222> (1)..(1)
   <223> Xaa = Glu or Gln
<220>
   <221> MISC_FEATURE
   <222> (7)..(7)
   <223> Xaa=Ser or Leu
<220>
   <221> MISC_FEATURE
   <222> (46)..(46)
   <223> Xaa=Glu, Val, Asp, or Ser
<220>
   <221> MISC_FEATURE
   <222> (56)..(56)
   <223> Xaa at position 56 is any amino acid, provided that if Xaa at position 57 is neither Asp nor Pro and Xaa at position 59 is Ser or Thr, then Xaa at position 56 is not Asn
<220>
   <221> MISC_FEATURE
   <222> (57)..(57)
   <223> Xaa at position 57 is any amino acid, provided that if Xaa at position 56 is Asn and Xaa at position 58 is Ser or Thr, then Xaa at position 57 is Asp or Pro
<220>
   <221> MISC_FEATURE
   <222> (58)..(58)
   <223> Xaa at position 58 is any amino acid, provided that if Xaa at position 56 is Asn and Xaa at position 57 is neither Asp nor Pro, then Xaa at position 58 is neither Ser nor Thr
<220>
   <221> MISC_FEATURE
   <222> (63)..(63)
   <223> Xaa=Thr or Ser
<220>
   <221> MISC_FEATURE
   <222> (75)..(75)
   <223> Xaa=Ala, Ser, Val, or Thr
<220>
   <221> MISC_FEATURE
   <222> (76)..(76)
   <223> Xaa=Lys or Arg
<220>
   <221> MISC_FEATURE
   <222> (89)..(89)
   <223> Xaa=Glu or Asp
<220>
   <221> MISC_FEATURE
   <222> (107)..(107)
   <223> Xaa=Leu or Thr
<400> 19
   Xaa Val Gln Leu Val Glu Xaa Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Ser Met Ser Trp Val Arg Gln Ala Pro Gly Lys Gly Leu Xaa Leu Val
           35                  40                  45
   Ala Gln Ile Asn Ser Val Gly Xaa Xaa Xaa Tyr Tyr Pro Asp Xaa Val
       50                  55                  60
   Lys Gly Arg Phe Thr Ile Ser Arg Asp Asn Xaa Xaa Asn Thr Leu Tyr
   65                  70                  75                  80
   Leu Gln Met Asn Ser Leu Arg Ala Xaa Asp Thr Ala Val Tyr Tyr Cys
                   85                  90                  95
   Ala Ser Gly Asp Tyr Trp Gly Gln Gly Thr Xaa Val Thr Val Ser Ser
               100                 105                 110
<210> 20
   <211> 112
   <212> PRT
   <213> Artificial
<220>
   <223> synthetic construct - humanized
<220>
   <221> MIST-FEATURE
   <222> (56)..(56)
   <223> xaa at position 56 is any amino acid, provided that if xaa at position 57 is neither Asp nor Pro and Xaa at position 59 is Ser or Thr, then xaa at position 56 is not Asn
<220>
   <221> MISC_FEATURE
   <222> (57)..(57)
   <223> xaa at position 57 is any amino acid, provided that if xaa at position 56 is Asn and xaa at position 58 is ser or Thr, then xaa at position 57 is Asp or Pro
<220>
   <221> MISC_FEATURE
   <222> (58)..(58)
   <223> xaa at position 58 is any amono acid, provided that if Xaa at position 56 is Asn and Xaa at position 57 is neither Asp nor Pro, then Xaa at position 58 is neither Ser nor Thr
<400> 20
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Ser Met Ser Trp Val Arg Gln Ala Pro Gly Lys Gly Leu Glu Leu Val
           35                  40                  45
   Ala Gln Ile Asn Ser Val Gly Xaa Xaa Xaa Tyr Tyr Pro Asp Thr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Ser Gly Asp Tyr Trp Gly Gln Gly Thr Leu Val Thr Val Ser Ser
               100                 105                 110
<210> 21
   <211> 442
   <212> PRT
   <213> Artificial
<220>
   <223> synthetic construct - humanized
<220>
   <221> MISC_FEATURE
   <222> (56)..(56)
   <223> xaa at position 56 is any amono acid, provided that if xaa at position 57 is neither Asp nor Pro and xaa at position 59 is ser or Thr, then xaa at position 56 is not Asn
<220>
   <221> MISC_FEATURE
   <222> (57)..(57)
   <223> Xaa at position 57 is any amono acid, provided that if xaa at position 56 is Asn and Xaa at position 58 is Ser or Thr, then Xaa at position 57 is Asp or Pro
<220>
   <221> MISC_FEATURE
   <222> (58)..(58)
   <223> xaa at position 58 is any amono acid, provided that if xaa at position 56 is Asn and xaa at position 57 is neither Asp nor Pro, then Xaa at position 58 is neither Ser nor Thr
<400> 21
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Arg Tyr
   20 25 30
   Ser Met Ser Trp Val Arg Gln Ala Pro Gly Lys Gly Leu Glu Leu Val
   35 40 45
   Ala Gln Ile Asn Ser Val Gly Xaa Xaa Xaa Tyr Tyr Pro Asp Thr Val
   50 55 60
   Lys Gly Arg Phe Thr Ile Ser Arg Asp Asn Ala Lys Asn Thr Leu Tyr
   65 70 75 80
   Leu Gln Met Asn Ser Leu Arg Ala Glu Asp Thr Ala Val Tyr Tyr Cys
   85 90 95
   Ala Ser Gly Asp Tyr Trp Gly Gln Gly Thr Leu Val Thr Val Ser Ser
   100 105 110
   Ala Ser Thr Lys Gly Pro Ser Val Phe Pro Leu Ala Pro Ser Ser Lys
   115 120 125
   Ser Thr Ser Gly Gly Thr Ala Ala Leu Gly Cys Leu Val Lys Asp Tyr
   130 135 140
   Phe Pro Glu Pro Val Thr Val Ser Trp Asn Ser Gly Ala Leu Thr Ser
   145 150 155 160
   Gly Val His Thr Phe Pro Ala Val Leu Gln Ser Ser Gly Leu Tyr Ser
   165 170 175
   Leu Ser Ser Val Val Thr Val Pro Ser Ser Ser Leu Gly Thr Gln Thr
   180 185 190
   Tyr Ile Cys Asn Val Asn His Lys Pro Ser Asn Thr Lys Val Asp Lys
   195 200 205
   Lys Val Glu Pro Lys Ser Cys Asp Lys Thr His Thr Cys Pro Pro Cys
   210 215 220
   Pro Ala Pro Glu Leu Leu Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser His Glu Asp Pro Glu Val Lys Phe Asn Trp
               260                 265                 270
   Tyr Val Asp Gly Val Glu Val His Asn Ala Lys Thr Lys Pro Arg Glu
           275                 280                 285
   Glu Gln Tyr Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Ala Leu Pro Ala Pro Ile Glu Lys Thr Ile Ser Lys Ala Lys Gly
                   325                 330                 335
   Gln Pro Arg Glu Pro Gln Val Tyr Thr Leu Pro Pro Ser Arg Asp Glu
               340                 345                 350
   Leu Thr Lys Asn Gln Val Ser Leu Thr Cys Leu Val Lys Gly Phe Tyr
           355                 360                 365
   Pro Ser Asp Ile Ala Val Glu Trp Glu Ser Asn Gly Gln Pro Glu Asn
       370                 375                 380
   Asn Tyr Lys Thr Thr Pro Pro Val Leu Asp Ser Asp Gly Ser Phe Phe
   385                 390                 395                 400
   Leu Tyr Ser Lys Leu Thr Val Asp Lys Ser Arg Trp Gln Gln Gly Asn
                   405                 410                 415
   Val Phe Ser Cys Ser Val Met His Glu Ala Leu His Asn His Tyr Thr
               420                 425                 430
   Gln Lys Ser Leu Ser Leu Ser Pro Gly Lys
           435                 440

### SEQUENCE LISTING

<110> Eli Lilly and Company
<120> METHOD FOR TREATING ANXIETY AND MOOD DISORDERS IN OLDER SUBJECTS
<130> X15667 National Stage
<140> 60/375462
   <141> 2002-04-25
<150> PCT/US03/10473
   <151> 2003-04-20
<160> 21
<170> PatentIn version 3.2
<210> 1
   <211> 16
   <212> PRT
   <213> Mus sp.
<400> 1
   Arg Ser Ser Gln Ser Leu Ile Tyr Ser Asp Gly Asn Ala Tyr Leu His
   1               5                   10                  15
<210> 2
   <211> 7
   <212> PRT
   <213> Mus sp.
<400> 2
   Lys Val Ser Asn Arg Phe Ser
   1               5
<210> 3
   <211> 9
   <212> PRT
   <213> Mus sp.
<400> 3
   Ser Gln Ser Thr His Val Pro Trp Thr
   1               5
<210> 4
   <211> 5
   <212> PRT
   <213> Mus sp.
<400> 4
   Arg Tyr Ser Met Ser
   1               5
<210> 5
   <211> 17
   <212> PRT
   <213> Mus sp.
<400> 5
   Gln Ile Asn Ser Val Gly Asn Ser Thr Tyr Tyr Pro Asp Thr Val Lys
   1               5                   10                  15
   Gly
<210> 6
   <211> 3
   <212> PRT
   <213> Mus sp.
<400> 6
   Gly Asp Tyr
   1
<210> 7
   <211> 113
   <212> PRT
   <213> Artificial Sequence
<220>
   <223> synthetic construct - humanized
<220>
   <221> MISC_FEATURE
   <222> (2)..(2)
   <223> Xaa=val or Ile
<220>
   <221> MISC_FEATURE
   <222> (7)..(7)
   <223> Xaa=Ser or Thr
<220>
   <221> MISC_FEATURE
   <222> (14)..(14)
   <223> Xaa=Thr or Ser
<220>
   <221> MISC_FEATURE
   <222> (15)..(15)
   <223> Xaa=Leu or Pro
<220>
   <221> MISC_FEATURE
   <222> (30)..(30)
   <223> Xaa=Ile or val
<220>
   <221> MISC_FEATURE
   <222> (50)..(50)
   <223> Xaa=Arg, Gln, or Lys
<220>
   <221> MISC_FEATURE
   <222> (88)..(88)
   <223> Xaa=Val or Leu
<220>
   <221> MISC_FEATURE
   <222> (105)..(105)
   <223> Xaa=Gln or Gly
<220>
   <221> MISC_FEATURE
   <222> (108)..(108)
   <223> Xaa=Lys or Arg
<220>
   <221> MISC_FEATURE
   <222> (109)..(109)
   <223> Xaa=val or Leu
<400> 7
   Asp Xaa Val Met Thr Gln Xaa Pro Leu Ser Leu Pro Val Xaa Xaa Gly
   1               5                   10                  15
   Gln Pro Ala Ser Ile Ser Cys Arg Ser Ser Gln Ser Leu Xaa Tyr Ser
               20                  25                  30
   Asp Gly Asn Ala Tyr Leu His Trp Phe Leu Gln Lys Pro Gly Gln Ser
           35                  40                  45
   Pro Xaa Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Xaa Gly Val Tyr Tyr Cys Ser Gln Ser
                   85                  90                  95
   Thr His Val Pro Trp Thr Phe Gly Xaa Gly Thr Xaa Xaa Glu Ile Lys
               100                 105                 110
   Arg
<210> 8
   <211> 112
   <212> PRT
   <213> Artificial Sequence
<220>
   <223> synthetic construct - humanized
<220>
   <221> MISC_FEATURE
   <222> (1)..(1)
   <223> Xaa=Glu or Gln
<220>
   <221> MISC_FEATURE
   <222> (7)..(7)
   <223> Xaa=Ser or Leu
<220>
   <221> MISC_FEATURE
   <222> (46)..(46)
   <223> xaa= Glu, Val, Asp, or Ser
<220>
   <221> MISC_FEATURE
   <222> (63)..(63)
   <223> Xaa= Thr or Ser
<220>
   <221> MISC_FEATURE
   <222> (75)..(75)
   <223> Xaa=Ala, Ser, Val, or Thr
<220>
   <221> MISC_FEATURE
   <222> (76)..(76)
   <223> Xaa=Lys or Arg
<220>
   <221> MISC_FEATURE
   <222> (89)..(89)
   <223> Xaa=Glu or Asp
<220>
   <221> MISC_FEATURE
   <222> (107)..(107)
   <223> Xaa=Leu or Thr
<400> 8
   Xaa Val Gln Leu Val Glu Xaa Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Ser Met Ser Trp Val Arg Gln Ala Pro Gly Lys Gly Leu Xaa Leu Val
           35                  40                  45
   Ala Gln Ile Asn Ser Val Gly Asn Ser Thr Tyr Tyr Pro Asp Xaa Val
       50                  55                  60
   Lys Gly Arg Phe Thr Ile Ser Arg Asp Asn Xaa Xaa Asn Thr Leu Tyr
   65                  70                  75                  80
   Leu Gln Met Asn Ser Leu Arg Ala Xaa Asp Thr Ala Val Tyr Tyr Cys
                   85                  90                  95
   Ala Ser Gly Asp Tyr Trp Gly Gln Gly Thr Xaa Val Thr Val Ser Ser
               100                 105                 110
<210> 9
   <211> 113
   <212> PRT
   <213> Artificial
<220>
   <223> Synthetic construct - humanized
<400> 9
   Asp Val Val Met Thr Gln Ser Pro Leu Ser Leu Pro Val Thr Leu Gly
   1               5                   10                  15
   Gln Pro Ala Ser Ile Ser Cys Arg Ser Ser Gln Ser Leu Ile Tyr Ser
               20                  25                  30
   Asp Gly Asn Ala Tyr Leu His Trp Phe Leu Gln Lys Pro Gly Gln Ser
           35                  40                  45
   Pro Arg Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ser Gln Ser
                   85                  90                  95
   Thr His Val Pro Trp Thr Phe Gly Gln Gly Thr Lys Val Glu Ile Lys
               100                 105                 110
   Arg
<210> 10
   <211> 112
   <212> PRT
   <213> Artificial
<220>
   <223> Synthetic construct - humanized
<400> 10
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Ser Met Ser Trp Val Arg Gln Ala Pro Gly Lys Gly Leu Glu Leu Val
           35                  40                  45
   Ala Gln Ile Asn Ser Val Gly Asn Ser Thr Tyr Tyr Pro Asp Thr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Ser Gly Asp Tyr Trp Gly Gln Gly Thr Leu Val Thr Val Ser Ser
               100                 105                 110
<210> 11
   <211> 219
   <212> PRT
   <213> Artificial
<220>
   <223> Synthetic construct - humanized
<400> 11
   Asp Val Val Met Thr Gln Ser Pro Leu Ser Leu Pro Val Thr Leu Gly
   1 5 10 15
   Gln Pro Ala Ser Ile Ser Cys Arg Ser Ser Gln Ser Leu Ile Tyr Ser
   20 25 30
   Asp Gly Asn Ala Tyr Leu His Trp Phe Leu Gln Lys Pro Gly Gln Ser
   35 40 45
   Pro Arg Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Ser Gln Ser
   85 90 95
   Thr His Val Pro Trp Thr Phe Gly Gln Gly Thr Lys Val Glu Ile Lys
   100 105 110
   Arg Thr Val Ala Ala Pro Ser Val Phe Ile Phe Pro Pro Ser Asp Glu
   115 120 125
   Gln Leu Lys Ser Gly Thr Ala Ser Val Val Cys Leu Leu Asn Asn Phe
   130 135 140
   Tyr Pro Arg Glu Ala Lys Val Gln Trp Lys Val Asp Asn Ala Leu Gln
   145 150 155 160
   Ser Gly Asn Ser Gln Glu Ser Val Thr Glu Gln Asp Ser Lys Asp Ser
   165 170 175
   Thr Tyr Ser Leu Ser Ser Thr Leu Thr Leu Ser Lys Ala Asp Tyr Glu
   180 185 190
   Lys His Lys Val Tyr Ala Cys Glu Val Thr His Gln Gly Leu Ser Ser
   195 200 205
   Pro Val Thr Lys Ser Phe Asn Arg Gly Glu Cys
   210 215
<210> 12
   <211> 442
   <212> PRT
   <213> Artificial
<220>
   <223> Synthetic construct - humanized
<400> 12
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Arg Tyr
   20 25 30
   Ser Met Ser Trp Val Arg Gln Ala Pro Gly Lys Gly Leu Glu Leu Val
   35 40 45
   Ala Gln Ile Asn Ser Val Gly Asn Ser Thr Tyr Tyr Pro Asp Thr Val
   50 55 60
   Lys Gly Arg Phe Thr Ile Ser Arg Asp Asn Ala Lys Asn Thr Leu Tyr
   65 70 75 80
   Leu Gln Met Asn Ser Leu Arg Ala Glu Asp Thr Ala Val Tyr Tyr Cys
   85 90 95
   Ala Ser Gly Asp Tyr Trp Gly Gln Gly Thr Leu Val Thr Val Ser Ser
   100 105 110
   Ala Ser Thr Lys Gly Pro Ser Val Phe Pro Leu Ala Pro Ser Ser Lys
   115 120 125
   Ser Thr Ser Gly Gly Thr Ala Ala Leu Gly Cys Leu Val Lys Asp Tyr
   130 135 140
   Phe Pro Glu Pro Val Thr Val Ser Trp Asn Ser Gly Ala Leu Thr Ser
   145 150 155 160
   Gly Val His Thr Phe Pro Ala Val Leu Gln Ser Ser Gly Leu Tyr Ser
   165 170 175
   Leu Ser Ser Val Val Thr Val Pro Ser Ser Ser Leu Gly Thr Gln Thr
   180 185 190
   Tyr Ile Cys Asn Val Asn His Lys Pro Ser Asn Thr Lys Val Asp Lys
   195 200 205
   Lys Val Glu Pro Lys Ser Cys Asp Lys Thr His Thr Cys Pro Pro Cys
   210 215 220
   Pro Ala Pro Glu Leu Leu Gly Gly Pro Ser Val Phe Leu Phe Pro Pro
   225 230 235 240
   Lys Pro Lys Asp Thr Leu Met Ile Ser Arg Thr Pro Glu Val Thr Cys
   245 250 255
   Val Val Val Asp Val Ser His Glu Asp Pro Glu Val Lys Phe Asn Trp
   260 265 270
   Tyr Val Asp Gly Val Glu Val His Asn Ala Lys Thr Lys Pro Arg Glu
   275 280 285
   Glu Gln Tyr Asn Ser Thr Tyr Arg Val Val Ser Val Leu Thr Val Leu
   290 295 300
   His Gln Asp Trp Leu Asn Gly Lys Glu Tyr Lys Cys Lys Val Ser Asn
   305 310 315 320
   Lys Ala Leu Pro Ala Pro Ile Glu Lys Thr Ile Ser Lys Ala Lys Gly
   325 330 335
   Gln Pro Arg Glu Pro Gln Val Tyr Thr Leu Pro Pro Ser Arg Asp Glu
   340 345 350
   Leu Thr Lys Asn Gln Val Ser Leu Thr Cys Leu Val Lys Gly Phe Tyr
   355 360 365
   Pro Ser Asp Ile Ala Val Glu Trp Glu Ser Asn Gly Gln Pro Glu Asn
   370 375 380
   Asn Tyr Lys Thr Thr Pro Pro Val Leu Asp Ser Asp Gly Ser Phe Phe
   385 390 395 400
   Leu Tyr Ser Lys Leu Thr Val Asp Lys Ser Arg Trp Gln Gln Gly Asn
   405 410 415
   Val Phe Ser Cys Ser Val Met His Glu Ala Leu His Asn His Tyr Thr
   420 425 430
   Gln Lys Ser Leu Ser Leu Ser Pro Gly Lys
   435 440
<210> 13
   <211> 17
   <212> PRT
   <213> Mus sp.
<220>
   <221> MISC_FEATURE
   <222> (7)..(7)
   <223> xaa at position 7 is any amino acid, provided that if xaa at position 8 is neither Asp nor Pro and xaa at position 9 is Ser or Thr, then xaa at position 7 is not Asn
<220>
   <221> MISC_FEATURE
   <222> (8)..(8)
   <223> xaa at position 8 is any amino acid, provided that if Xaa at position 7 is Asn and xaa at position 9 is Ser or Thr, then xaa at position 8 is Asp or Pro
<220>
   <221> MISC_FEATURE
   <222> (9)..(9)
   <223> Xaa at position 9 is any amino acid, provided that if Xaa at position 7 is Asn and Xaa at position 8 is neither Asp nor Pro, then xaa at position 9 is neither Ser nor Thr
<400> 13
   Gln Ile Asn Ser Val Gly Xaa Xaa Xaa Tyr Tyr Pro Asp Thr Val Lys
   1               5                   10                  15
   Gly
<210> 14
   <211> 17
   <212> PRT
   <213> Mus sp.
<220>
   <221> MISC_FEATURE
   <222> (7)..(7)
   <223> xaa at position 7 is selected from the group consisting of Ala, Cys, Asp, Glu, Phe, Gly, His, Ile, Lys, Leu, Met, Pro, Gln, Arg, Ser, Thr, Val, Trp, and Tyr
<220>
   <221> MISC_FEATURE
   <222> (8)..(8)
   <223> xaa at position 8 is selected from the group consisting of Ala, Cys, Asp, Glu, Phe, Gly, His, Ile, Lys, Leu, Met, Asn, Pro, Gln, Arg, Ser, Thr, Val, Trp, and Tyr
<220>
   <221> MISC_FEATURE
   <222> (9)..(9)
   <223> xaa at position 9 is selected from the group consisting of Ala, Cys, Asp, Glu, Phe, Gly, His, Ile, Lys, Leu, Met, Asn, Pro, Gln, Arg, Ser, Thr, Val, Trp, and Tyr
<400> 14
<210> 15
   <211> 17
   <212> PRT
   <213> Mus sp.
<220>
   <221> MISC_FEATURE
   <222> (7)..(7)
   <223> Xaa=Asn
<220>
   <221> MISC_FEATURE
   <222> (8)..(8)
   <223> Xaa at position 8 is selected from the group consisting of Ala, Cys, Asp, Glu, Phe, Gly, His, Ile, Lys, Leu, Met, Asn, Pro, Gln, Arg, Ser, Thr, Val, Trp, and Tyr
<220>
   <221> MISC_FEATURE
   <222> (9)..(9)
   <223> Xaa at position 9 is selected from the group consisting of Ala, Cys, Asp, Glu, Phe, Gly, His, Ile, Lys, Leu, Met, Asn, Pro, Gln, Arg, Ser, Thr, Val, Trp, and Tyr
<400> 15
   Gln Ile Asn Ser Val Gly Xaa Xaa Xaa Tyr Tyr Pro Asp Thr Val Lys
   1               5                   10                  15
   Gly
<210> 16
   <211> 17
   <212> PRT
   <213> Mus sp.
<220>
   <221> MISC_FEATURE
   <222> (7)..(7)
   <223> Xaa=Asn
<220>
   <221> MISC_FEATURE
   <222> (8)..(8)
   <223> Xaa at position 8 is selected from the group consisting of Asp and Pro
<220>
   <221> MISC_FEATURE
   <222> (9)..(9)
   <223> Xaa at position 9 is selected from the group consisting of Ser and Thr
<400> 16
   Gln Ile Asn Ser Val Gly Xaa Xaa Xaa Tyr Tyr Pro Asp Thr Val Lys
   1               5                   10                  15
   Gly
<210> 17
   <211> 17
   <212> PRT
   <213> Mus sp.
<220>
   <221> MISC_FEATURE
   <222> (7)..(7)
   <223> Xaa at position 7 is selected from the group consisting of Ala, Gly, His, Gln, Ser, and Thr
<220>
   <221> MISC_FEATURE
   <222> (8)..(8)
   <223> Xaa at position 8 is selected from the group consisting of Ala, Gly, His, Asn, Gln, Ser, and Thr
<220>
   <221> MISC_FEATURE
   <222> (9)..(9)
   <223> Xaa at position 9 is selected from the group consisting of Ala, Gly, His, Asn, Gln, Ser, and Thr
<400> 17
   Gln Ile Asn Ser Val Gly Xaa Xaa Xaa Tyr Tyr Pro Asp Thr Val Lys
   1               5                   10                  15
   Gly
<210> 18
   <211> 17
   <212> PRT
   <213> Mus sp.
<220>
   <221> MISC_FEATURE
   <222> (7)..(7)
   <223> Xaa at position 7 is Asn
<220>
   <221> MISC_FEATURE
   <222> (8)..(8)
   <223> Xaa at position 8 is selected from the group consisting of Ala, Gly, His, Asn, Gln, Ser, and Thr
<220>
   <221> MISC_FEATURE
   <222> (9)..(9)
   <223> Xaa at position 9 is selected from the group consisting of Ala, Gly, His, Asn, and Gln
<400> 18
   Gln Ile Asn Ser Val Gly Xaa Xaa Xaa Tyr Tyr Pro Asp Thr Val Lys
   1               5                   10                  15
   Gly
<210> 19
   <211> 112
   <212> PRT
   <213> Artificial
<220>
   <223> synthetic construct - humanized
<220>
   <221> MISC_FEATURE
   <222> (1)..(1)
   <223> Xaa = Glu or Gln
<220>
   <221> MISC_FEATURE
   <222> (7)..(7)
   <223> Xaa=Ser or Leu
<220>
   <221> MISC_FEATURE
   <222> (46)..(46)
   <223> Xaa=Glu, Val, Asp, or Ser
<220>
   <221> MISC_FEATURE
   <222> (56)..(56)
   <223> Xaa at position 56 is any amino acid, provided that if Xaa at position 57 is neither Asp nor Pro and xaa at position 59 is Ser or Thr, then xaa at position 56 is not Asn
<220>
   <221> MISC_FEATURE
   <222> (57)..(57)
   <223> Xaa at position 57 is any amino acid, provided that if xaa at position 56 is Asn and Xaa at position 58 is Ser or Thr, then Xaa at position 57 is Asp or Pro
<220>
   <221> MISC_FEATURE
   <222> (58)..(58)
   <223> xaa at position 58 is any amino acid, provided that if xaa at position 56 is Asn and xaa at position 57 is neither Asp nor Pro, then xaa at position 58 is neither Ser nor Thr
<220>
   <221> MISC_FEATURE
   <222> (63)..(63)
   <223> Xaa=Thr or Ser
<220>
   <221> MISC_FEATURE
   <222> (75)..(75)
   <223> Xaa=Ala, Ser, val, or Thr
<220>
   <221> MISC_FEATURE
   <222> (76)..(76)
   <223> Xaa=Lys or Arg
<220>
   <221> MISC_FEATURE
   <222> (89)..(89)
   <223> Xaa=Glu or Asp
<220>
   <221> MISC_FEATURE
   <222> (107)..(107)
   <223> Xaa=Leu or Thr
<400> 19
   Xaa Val Gln Leu Val Glu Xaa Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Ser Met Ser Trp Val Arg Gln Ala Pro Gly Lys Gly Leu Xaa Leu Val
           35                  40                  45
   Ala Gln Ile Asn Ser Val Gly Xaa Xaa Xaa Tyr Tyr Pro Asp Xaa Val
       50                  55                  60
   Lys Gly Arg Phe Thr Ile Ser Arg Asp Asn Xaa Xaa Asn Thr Leu Tyr
   65                  70                  75                  80
   Leu Gln Met Asn Ser Leu Arg Ala Xaa Asp Thr Ala Val Tyr Tyr Cys
                   85                  90                  95
   Ala Ser Gly Asp Tyr Trp Gly Gln Gly Thr Xaa Val Thr Val Ser Ser
               100                 105                 110
<210> 20
   <211> 112
   <212> PRT
   <213> Artificial
<220>
   <223> synthetic construct - humanized
<220>
   <221> MISC_FEATURE
   <222> (56)..(56)
   <223> Xaa at position 56 is any amino acid, provided that if Xaa at position 57 is neither Asp nor Pro and Xaa at position 59 is ser or Thr, then xaa at position 56 is not Asn
<220>
   <221> MISC_FEATURE
   <222> (57)..(57)
   <223> Xaa at position 57 is any amino acid, provided that if Xaa at position 56 is Asn and Xaa at position 58 is Ser or Thr, then xaa at position 57 is Asp or Pro
<220>
   <221> MISC_FEATURE
   <222> (58)..(58)
   <223> Xaa at position 58 is any amono acid, provided that if Xaa at position 56 is Asn and Xaa at position 57 is neither Asp nor Pro, then Xaa at position 58 is neither Ser nor Thr
<400> 20
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Ser Met Ser Trp Val Arg Gln Ala Pro Gly Lys Gly Leu Glu Leu Val
           35                  40                  45
   Ala Gln Ile Asn Ser Val Gly Xaa Xaa Xaa Tyr Tyr Pro Asp Thr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Ser Gly Asp Tyr Trp Gly Gln Gly Thr Leu Val Thr Val Ser Ser
               100                 105                 110
<210> 21
   <211> 442
   <212> PRT
   <213> Artificial
<220>
   <223> synthetic construct - humanized
<220>
   <221> MISC_FEATURE
   <222> (56)..(56)
   <223> Xaa at position 56 is any amono acid, provided that if Xaa at position 57 is neither Asp nor Pro and Xaa at position 59 is Ser or Thr, then xaa at position 56 is not Asn
<220>
   <221> MISC_FEATURE
   <222> (57)..(57)
   <223> Xaa at position 57 is any amono acid, provided that if Xaa at position 56 is Asn and Xaa at position 58 is Ser or Thr, then xaa at position 57 is Asp or Pro
<220>
   <221> MISC_FEATURE
   <222> (58)..(58)
   <223> Xaa at position 58 is any amono acid, provided that if Xaa at position 56 is Asn and Xaa at position 57 is neither Asp nor Pro, then Xaa at position 58 is neither Ser nor Thr
<400> 21
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Ser Met Ser Trp Val Arg Gln Ala Pro Gly Lys Gly Leu Glu Leu Val
           35                  40                  45
   Ala Gln Ile Asn Ser Val Gly Xaa Xaa Xaa Tyr Tyr Pro Asp Thr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Ser Gly Asp Tyr Trp Gly Gln Gly Thr Leu Val Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Lys Thr His Thr Cys Pro Pro Cys
       210                 215                 220
   Pro Ala Pro Glu Leu Leu Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser His Glu Asp Pro Glu Val Lys Phe Asn Trp
               260                 265                 270
   Tyr Val Asp Gly Val Glu Val His Asn Ala Lys Thr Lys Pro Arg Glu
           275                 280                 285
   Glu Gln Tyr Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Ala Leu Pro Ala Pro Ile Glu Lys Thr Ile Ser Lys Ala Lys Gly
                   325                 330                 335
   Gln Pro Arg Glu Pro Gln Val Tyr Thr Leu Pro Pro Ser Arg Asp Glu
               340                 345                 350
   Leu Thr Lys Asn Gln Val Ser Leu Thr Cys Leu Val Lys Gly Phe Tyr
           355                 360                 365
   Pro Ser Asp Ile Ala Val Glu Trp Glu Ser Asn Gly Gln Pro Glu Asn
       370                 375                 380
   Asn Tyr Lys Thr Thr Pro Pro Val Leu Asp Ser Asp Gly Ser Phe Phe
   385                 390                 395                 400
   Leu Tyr Ser Lys Leu Thr Val Asp Lys Ser Arg Trp Gln Gln Gly Asn
                   405                 410                 415
   Val Phe Ser Cys Ser Val Met His Glu Ala Leu His Asn His Tyr Thr
               420                 425                 430
   Gln Lys Ser Leu Ser Leu Ser Pro Gly Lys
           435                 440